(19) Europäisches Patentamt / European Patent Office / Office européen des brevets

(11) **EP 4 431 618 A1**

(12) **EUROPEAN PATENT APPLICATION**
published in accordance with Art. 153(4) EPC

(43) Date of publication:
**18.09.2024 Bulletin 2024/38**

(21) Application number: **22892128.4**

(22) Date of filing: **11.11.2022**

(51) International Patent Classification (IPC):
***C12Q 1/6886*** (2018.01)

(52) Cooperative Patent Classification (CPC):
**C12Q 1/6886; G16B 30/00; G16B 40/00;
G16H 50/20; G16H 50/30; G16H 50/70;** Y02A 90/10

(86) International application number:
**PCT/CN2022/131540**

(87) International publication number:
**WO 2023/083335 (19.05.2023 Gazette 2023/20)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **12.11.2021 CN 202111342507**

(71) Applicant: **Lisen Imprinting Diagnostics (Wuxi),
Co., Ltd.**
**Wuxi, Jiangsu 214000 (CN)**

(72) Inventors:
• **CHENG, Tong**
**Wuxi, Jiangsu 214000 (CN)**
• **ZHOU, Ning**
**Wuxi, Jiangsu 214000 (CN)**

(74) Representative: **Lavoix**
**Bayerstraße 83**
**80335 München (DE)**

(54) **GRADING MODEL FOR DETECTING BENIGN AND MALIGNANT DEGREE OF TUMOR AND AN APPLICATION THEREOF**

(57) A grading model for detecting the benign and malignant degree of a tumor and an application thereof. A detection model is used for determining the benign and malignant degree of a tumor by intuitively observing a change of an imprinted gene of the tumor at an early stage at a single-cell and tissue level. Moreover, the type and the benign and malignant degree of the tumor can be accurately determined by combining expression changes of imprinted and non-imprinted genes.

Fig. 1

**Description**

**PRIORITY INFORMATION**

This application claims priority to and the benefit of Patent Application No. 202111342507.1, filed to the China National Intellectual Property Administration on November 12, 2021, which is incorporated herein by reference in its entirety.

**FIELD**

The present disclosure relates to the field of biotechnology and the field of genetic diagnosis, and particularly, to a grading model for detecting a benign and malignant degree of a tumor and uses thereof.

**BACKGROUND**

Malignant tumors are one of the major factors that seriously threaten human health. There are 14 million new cancer patients worldwide every year, and 8.2 million patients die of cancer at the same time. Unlimited proliferation and metastasis are the main characteristics of cancer cells. Most cancer patients eventually die due to complications such as organ failure, caused by the extensive and rapid metastasis of cancer cells. Early stage cancer has small lesions, less surgical trauma, and fewer cancer cells entering the circulatory system, and thus it cannot form stable metastases. Therefore, timely and effective treatment in the early stage is of great significance to prevent recurrence and prolong life. The current diagnostic criteria for cancer are the cell and tissue morphology of the tumor. However, morphological observation requires very high experience of pathologists. At the same time, early cancers have not yet undergone typical morphological changes, which may easily lead to misdiagnosis. Since changes in molecular biology such as epigenetics and genetics are earlier than morphological changes in the process of cancer development, early cancers can be detected more sensitively. Therefore, molecular markers are of great significance for early diagnosis of cancer.

Genomic imprinting is a mode of gene regulation in epigenetics characterized by the methylation of alleles from a specific parent, such that only one allele of a certain gene is expressed, while the other is trapped in a gene-silencing status. Such a gene is referred to as an imprinted (marked) gene. Loss of imprinting is an epigenetic change in which demethylation of an imprinted gene results in activation of the silent allele and initiation of gene expression. Numerous studies have shown that loss of imprinting is common in various cancers and occurs earlier than changes in cell and tissue morphology. Moreover, in healthy cells, the proportion of loss of imprinting is extremely low, in sharp contrast to cancer cells. Therefore, the methylation status of imprinted genes can be used as a pathological marker, and the abnormal status of cells can be analyzed through specific molecular detection techniques.

Since the functions of imprinted genes cover multiple aspects such as cell signaling, cell cycle regulation, intracellular and extracellular material transport, and extracellular matrix formation, the functions of imprinted genes in different cancers are different, and their expressions also vary greatly, thereby forming different sensitivities and specificities, which plays a huge role in invasion and metastasis in tumor development and prognosis. Moreover, the combination of different imprinted genes can also distinguish the type of tumor.

Based on the above reasons, no highly sensitive diagnostic marker is currently available for the early diagnosis of tumors. It is urgent to develop a new diagnostic marker, a diagnostic model, and a diagnostic device, for providing more accurate pre-diagnosis and diagnostic information and guiding the selection of treatment regimens by analyzing a change of a molecular marker of a tumor on the cellular level based on a biopsy sample derived from a patient.

**SUMMARY**

The present disclosure is intended to solve at least one of the technical problems in the related art to some extent. For this purpose, the present disclosure provides a grading model for detecting a benign and malignant degree of a tumor and uses thereof. The detection model is used for determining the benign and malignant degree of a tumor by intuitively observing a change of imprinted genes of the tumor at an early stage at a single-cell and tissue level. At the same time, by combining expression changes of imprinted and non-imprinted genes, the benign and malignant degree of the tumor can be more accurately determined, and the cancer types can be further distinguished.

Epigenetic changes such as DNA demethylation and histone acetylation occur in imprinted genes at the earliest stage of cancer, resulting in the reactivation of normally silent genes in a pair of alleles of imprinted genes. Thus, these two alleles are expression, which is referred to as loss of imprinting. Some imprinted and non-imprinted genes also undergo gene amplification during cancer development, with three or more gene copies, referred to as copy number abnormality. The loss of imprinting of the imprinted genes and the copy number abnormality of the imprinted and non-imprinted genes can be used for early molecular diagnosis of cancer.

For this purpose, in a first aspect, the present disclosure provides a grading model of gene expression status for a tumor. According to an embodiment of the present disclosure, the model includes grading an expression status of imprinted genes by calculating changes in a total expression, single-allelic expression, bi-allelic expression, and multi-allelic expression of the imprinted genes in the tumor. The imprinted genes include Z16, Z19, and Z21. The imprinted gene Z16 is SNRPN/SNURF, the imprinted gene Z19 is HM13, and the imprinted gene Z21 is NHP2L1.

According to an embodiment of the present disclosure, the imprinted genes further include any one or more of Z1, Z3, Z5, Z6, Z8, Z9, Z10, Z11, Z12, Z13, Z14, Z18, Z20, Z22, or Z28. The imprinted gene Z1 is GNAS, the imprinted gene Z3 is PEG10, the imprinted gene Z5 is MEST, the imprinted gene Z6 is PLAGL1, the imprinted gene Z8 is DCN, the imprinted gene Z9 is DLK1, the imprinted gene Z10 is GATM, the imprinted gene Z11 is GRB10, the imprinted gene Z12 is PEG3, the imprinted gene Z13 is SGCE, the imprinted gene Z14 is SLC38A4, the imprinted gene Z18 is GATA3, the imprinted gene Z20 is KCNQ1, the imprinted gene Z22 is PON2, and the imprinted gene Z28 is ZIC1.

According to an embodiment of the present disclosure, formulas for calculating the total expression, single-allelic expression, bi-allelic expression, and multi-allelic expression of the imprinted genes are as follows:

total expression = (b+c+d)/(a+b+c+d);
single-allelic expression = b/(b+c+d);
bi-allelic expression = c/(b+c+d);
multi-allelic expression = d/(b+c+d), where:
a denotes the number of cells in which no marker is present in a cell nucleus and the imprinted genes are not expressed; b denotes the number of cells in which one imprinted gene marker is present in the cell nucleus; c denotes the number of cells in which two imprinted gene markers are present in the cell nucleus; and d denotes the number of cells in which more than two imprinted gene markers are present in the cell nucleus.

According to an embodiment of the present disclosure, a bi-allelic expression of the imprinted gene Z16 is divided into five different grades:

Grade 0: the bi-allelic expression of the imprinted gene Z16 is lower than 15%;
Grade I: the bi-allelic expression of the imprinted gene Z16 ranges from 15% to 20%;
Grade II: the bi-allelic expression of the imprinted gene Z16 ranges from 20% to 26%;
Grade III: the bi-allelic expression of the imprinted gene Z16 ranges from 26% to 30%; and
Grade IV: the bi-allelic expression of the imprinted gene Z16 is greater than 30%.

A multi-allelic expression of the imprinted gene Z16 is divided into five different grades:

Grade 0: the multi-allelic expression of the imprinted gene Z16 is lower than 1.4%;
Grade I: the multi-allelic expression of the imprinted gene Z16 ranges from 1.4% to 3.4%;
Grade II: the multi-allelic expression of the imprinted gene Z16 ranges from 3.4% to 5.5%;
Grade III: the multi-allelic expression of the imprinted gene Z16 ranges from 5.5% to 9%; and
Grade IV: the multi-allelic expression of the imprinted gene Z16 is greater than 9%.

A total expression of the imprinted gene Z16 is divided into two different grades:

Grade 0: the total expression of the imprinted gene Z16 is lower than 17%; and
Grade I: the total expression of the imprinted gene Z16 is greater than or equal to 17%.

According to an embodiment of the present disclosure, a bi-allelic expression of the imprinted gene Z19 is divided into five different grades:

Grade 0: the bi-allelic expression of the imprinted gene Z19 is lower than 12%;
Grade I: the bi-allelic expression of the imprinted gene Z19 ranges from 12% to 20%;
Grade II: the bi-allelic expression of the imprinted gene Z19 ranges from 20% to 26%;
Grade III: the bi-allelic expression of the imprinted gene Z19 ranges from 26% to 30%; and
Grade IV: the bi-allelic expression of the imprinted gene Z19 is greater than 30%.

A multi-allelic expression of the imprinted gene Z19 is divided into five different grades:

Grade 0: the multi-allelic expression of the imprinted gene Z19 is lower than 1.4%;
Grade I: the multi-allelic expression of the imprinted gene Z19 ranges from 1.4% to 3.4%;

Grade II: the multi-allelic expression of the imprinted gene Z19 ranges from 3.4% to 6.3%;
Grade III: the multi-allelic expression of the imprinted gene Z19 ranges from 6.3% to 9%; and
Grade IV: the multi-allelic expression of the imprinted gene Z19 is greater than 9%.

A total expression of the imprinted gene Z19 is divided into two different grades:

Grade 0: the total expression of the imprinted gene Z19 is lower than 11%; and
Grade I: the total expression of the imprinted gene Z19 is greater than or equal to 11%.

According to an embodiment of the present disclosure, a bi-allelic expression of the imprinted gene Z21 is divided into five different grades:

Grade 0: the bi-allelic expression of the imprinted gene Z21 is lower than 12%;
Grade I: the bi-allelic expression of the imprinted gene Z21 ranges from 12% to 16%;
Grade II: the bi-allelic expression of the imprinted gene Z21 ranges from 16% to 20%;
Grade III: the bi-allelic expression of the imprinted gene Z21 ranges from 20% to 25%; and
Grade IV: the bi-allelic expression of the imprinted gene Z21 is greater than 25%.

A multi-allelic expression of the imprinted gene Z21 is divided into five different grades:

Grade 0: the multi-allelic expression of the imprinted gene Z21 is lower than 1.3%;
Grade I: the multi-allelic expression of the imprinted gene Z21 ranges from 1.3% to 3.2%;
Grade II: the multi-allelic expression of the imprinted gene Z21 ranges from 3.4% to 4.9%;
Grade III: the multi-allelic expression of the imprinted gene Z21 ranges from 4.9% to 6.2%; and
Grade IV: the multi-allelic expression of the imprinted gene Z21 is greater than 6.2%.

A total expression of the imprinted gene Z21 is divided into two different grades:

Grade 0: the total expression of the imprinted gene Z21 is lower than 14%; and
Grade I: the total expression of the imprinted gene Z21 is greater than or equal to 14%.

According to an embodiment of the present disclosure, a positive degree of the imprinted genes Z16, Z19, and Z21 is divided into negative, positive potential, low positive, moderate positive, and high positive; when the total expression of the imprinted gene is Grade 0, or when the total expression of the imprinted gene is Grade I as well as the bi-allelic expression and multi-allelic expression are both Grade 0, the positive degree of the imprinted gene is determined to be negative; when he total expression of the imprinted gene is Grade I as well as at least one of the bi-allelic expression and multi-allelic expression is Grade I and both are not Grade II, the positive degree of the imprinted gene is determined to be positive potential; when the total expression of the imprinted gene is Grade I as well as at least one of the bi-allelic expression and multi-allelic expression is Grade II and both are not Grade III, the positive degree of the imprinted gene is determined to be low positive; when the total expression of the imprinted gene is Grade I as well as at least one of the bi-allelic expression and multi-allelic expression is Grade III and both are not Grade IV, the positive degree of the imprinted gene is determined to be moderate positive; and when the total expression of the imprinted gene is Grade I as well as at least one of the bi-allelic expression and multi-allelic expression is Grade IV, the positive degree of the imprinted gene is determined to be high positive. The positive degree of the imprinted genes Z16, Z19, and Z21 are independent.

According to an embodiment of the present disclosure, a benign and malignant degree of the tumor is divided into benign tumor, cancer potential, early cancer, intermediate cancer, and terminal cancer; when the positive degree of the imprinted genes Z16, Z19, and Z21 are all negative, or when the positive degree of no more than one of the imprinted genes Z16, Z19, and Z21 is positive potential, the benign and malignant degree of the tumor is determined to be benign tumor; when the positive degree of at least two of the imprinted genes Z16, Z19, and Z21 are positive potential, or when the positive degree of no more than one of the imprinted genes Z16, Z19, and Z21 is low positive, the benign and malignant degree of the tumor is determined to be cancer potential; when the positive degree of at least two of the imprinted genes Z16, Z19, and Z21 is low positive, or when the positive degree of no more than one of the imprinted genes Z16, Z19, and Z21 is moderate positive, the benign and malignant degree of the tumor is determined to be early cancer; when the positive degree of at least two of the imprinted genes Z16, Z19, and Z21 are moderate positive, or when the positive degree of no more than one of the imprinted genes Z16, Z19, and Z21 is high positive, the benign and malignant degree of the tumor is determined to be intermediate cancer; and when the positive degree of at least two of the imprinted genes Z16, Z19, and Z21 is high positive, the benign and malignant degree of the tumor is determined to be terminal cancer.

In clinical applications, a sample determined to be benign tumor or cancer potential based on the benign and malignant degree of the tumor can be regarded as a benign sample; and a sample determined to be early cancer, intermediate cancer, or terminal cancer based on the benign and malignant degree of the tumor can be regarded as a malignant samples.

The combination of the imprinted genes Z16, Z19, and Z21 can be further combined with imprinted genes Z1, Z3, Z5, Z6, Z8, Z9, Z10, Z11, Z12, Z13, Z14, Z18, Z20, Z22, and Z28 to improve the accuracy of determining benign and malignant tumors.

According to an embodiment of the present disclosure, when a positive degree of the imprinted genes Z16, Z19, and Z21 are all negative or positive potential, or when the positive degree of no more than one of the imprinted genes Z16, Z19, and Z21 is low positive as well as a positive degree of the imprinted genes Z1, Z3, Z5, Z6, Z8, Z9, Z10, Z11, Z12, Z13, Z14, Z18, Z20, Z22, and Z28 are all negative, the benign and malignant degree of the tumor is determined to be a benign tumor; and when the positive degree of no more than one of the imprinted genes Z16, Z19, and Z21 is low positive as well as the positive degree of at least one of the imprinted genes Z1, Z3, Z5, Z6, Z8, Z9, Z10, Z11, Z12, Z13, Z14, Z18, Z20, Z22, and Z28 is positive, or when the positive degree of at least two of the imprinted genes Z16, Z19, and Z21 is low positive, or when the positive degree of at least one of the imprinted genes Z16, Z19, and Z21 is moderate positive, the benign and malignant degree of the tumor is determined to be a malignant tumor.

According to an embodiment of the present disclosure, the tumor is selected from thyroid cancer, lung cancer, bladder cancer, prostate cancer, skin cancer, breast cancer, cervical cancer, intestinal cancer, stomach cancer, esophageal cancer, pancreatic cancer, and liver cancer.

According to an embodiment of the present disclosure, the model further includes grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes BRAF and/or MYC.

When a positive degree of the imprinted genes Z16, Z19, and Z21 are all positive potential or below, or when the positive degree of only one of the imprinted genes Z16, Z19, and Z21 is low positive or above and the positive degree of the others of the imprinted genes Z16, Z19, and Z21 is negative or positive potential as well as the non-imprinted genes BRAF and MYC are both negative, a benign and malignant degree of the tumor is determined to be a benign tumor.

When the positive degree of only one of the imprinted genes Z16, Z19, and Z21 is low positive or above and the positive degree of the others of the imprinted genes Z16, Z19, and Z21 is negative or positive potential as well as at least one of the non-imprinted genes BRAF and MYC is positive, or when the positive degree of at least two of the imprinted genes Z16, Z19, and Z21 is low positive or above, the benign and malignant degree of the tumor is determined to be a malignant tumor.

The non-imprinted genes BRAF and MYC being negative means that the expression of the non-imprinted genes BRAF and MYC in a test sample is not different from that in a normal sample. The non-imprinted genes BRAF and MYC being positive means that the expression of the non-imprinted genes BRAF and MYC in the test sample is increased compared to that in the normal sample.

By adding the non-imprinted genes BRAF and/or MYC to the combination of imprinted genes, the detection accuracy of the model can be improved. In the present embodiment, the combination of imprinted genes Z16, Z19, and Z21 has a sensitivity of 96% and a specificity of 91% for determining thyroid tumors.

According to an embodiment of the present disclosure, the model further includes grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes HER2 and/or MYC.

When a positive degree of the imprinted genes Z16, Z19, and Z21 are all positive potential or below, or when the positive degree of only one of the imprinted genes Z16, Z19, and Z21 is low positive or above and the positive degree of the others of the imprinted genes Z16, Z19, and Z21 is negative or positive potential as well as the non-imprinted genes HER2 and MYC are both negative, the benign and malignant degree of the tumor is determined to be a benign tumor.

When the positive degree of only one of the imprinted genes Z16, Z19, and Z21 is low positive or above and the positive degree of the others of the imprinted genes Z16, Z19, and Z21 is negative or positive potential as well as at least one of the non-imprinted genes HER2 and MYC is positive, or when the positive degree of at least two of the imprinted genes Z16, Z19, and Z21 is low positive or above, the benign and malignant degree of the tumor is determined to be a malignant tumor.

The non-imprinted genes HER2 and MYC being negative means that the expression of the non-imprinted genes HER2 and MYC in a test sample is not different from that in a normal sample. The non-imprinted genes HER2 and MYC being positive means that the expression of the non-imprinted genes HER2 and MYC in the test sample is increased compared to that in the normal sample.

By adding the non-imprinted genes HER2 and/or MYC to the combination of imprinted genes, the detection accuracy of the model can be improved. In the present embodiment, the combination of imprinted genes Z16, Z19, and Z21 has a sensitivity of 98% and a specificity of 95% for determining lung tumors.

According to an embodiment of the present disclosure, the model further includes grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes GRP and/or SYP.

When a benign and malignant degree of the tumor is determined to be an early cancer or above in combination with the imprinted genes Z16, Z19, and Z21, and when at least one of the non-imprinted genes GRP and SYP is positive, a type of the tumor is determined to be small cell carcinoma.

When the benign and malignant degree of the tumor is determined to be early cancer or above in combination with the imprinted genes Z16, Z19, and Z21, and when the non-imprinted genes GRP and SYP are both negative, the type of the tumor is determined to be non-small cell carcinoma.

The non-imprinted genes GRP and SYP being negative means that the expression of the non-imprinted genes GRP and SYP in a test sample is not different from that in a normal sample. The non-imprinted genes GRP and SYP being positive means that the expression of the non-imprinted genes GRP and SYP in the test sample is increased compared to that in the normal sample.

By adding the non-imprinted genes GRP and/or SYP to the combination of imprinted genes, the type of the tumor can be further identified while the tumor is determined to be a malignant tumor. In the presented example, when the benign and malignant degree of the tumor is determined to be early cancer or above based on the combination of imprinted genes Z16, Z19, and Z21, by further adding the non-imprinted genes GRP and SYP, the identification accuracy for small cell carcinoma of lung and non-small cell carcinoma of lung is 100%.

According to an embodiment of the present disclosure, the model further includes grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes CDKN2A and/or MYC.

When a positive degree of the imprinted genes Z16, Z19, and Z21 are all positive potential or below, or when the positive degree of only one of the imprinted genes Z16, Z19, and Z21 is low positive or above and the positive degree of the others of the imprinted genes Z16, Z19, and Z21 is negative or positive potential as well as the non-imprinted genes CDKN2A and MYC are both negative, a benign and malignant degree of the tumor is determined to be a benign tumor.

When the positive degree of only one of the imprinted genes Z16, Z19, and Z21 is low positive or above and the positive degree of the others of the imprinted genes Z16, Z19, and Z21 is negative or positive potential as well as at least one of the non-imprinted genes CDKN2A and MYC is positive, or when the positive degree of at least two of the imprinted genes Z16, Z19, and Z21 is low positive or above, the benign and malignant degree of the tumor is determined to be a malignant tumor.

The non-imprinted genes CDKN2A and MYC being negative means that the expression of the non-imprinted genes CDKN2A and MYC in a test sample is not different from that in a normal sample. The non-imprinted genes CDKN2A and MYC being positive means that the expression of the non-imprinted genes CDKN2A and MYC in the test sample is increased compared to that in the normal sample.

By adding the non-imprinted genes CDKN2A and/or MYC to the combination of imprinted genes, the detection accuracy of the model can be improved. In the present embodiment, the combination of the imprinted genes Z16, Z19, and Z21 has a sensitivity of 96% and a specificity of 97% for determining bladder tumors.

According to an embodiment of the present disclosure, the model further includes grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes HER2 and/or BRAF.

When a positive degree of the imprinted genes Z16, Z19, and Z21 are all positive potential or below, or when the positive degree of only one of the imprinted genes Z16, Z19, and Z21 is low positive or above and the positive degree of the others of the imprinted genes Z16, Z19, and Z21 is negative or positive potential as well as the non-imprinted genes HER2 and BRAF are both negative, a benign and malignant degree of the tumor is determined to be a benign tumor.

When the positive degree of only one of the imprinted genes Z16, Z19, and Z21 is low positive or above and the positive degree of the others of the imprinted genes Z16, Z19, and Z21 is negative or positive potential as well as at least one of the non-imprinted genes HER2 and BRAF is positive, or when the positive degree of at least two of the imprinted genes Z16, Z19, and Z21 is low positive or above, the benign and malignant degree of the tumor is determined to be a malignant tumor.

The non-imprinted genes HER2 and BRAF being negative means that the expression of the non-imprinted genes HER2 and BRAF in a test sample is not different from that in a normal sample. The non-imprinted genes HER2 and BRAF being positive means that the expression of the non-imprinted genes HER2 and BRAF in the test sample is increased compared to that in the normal sample.

By adding the non-imprinted genes HER2 and/or BRAF to the combination of imprinted genes, the detection accuracy of the model can be improved. In the present embodiment, the combination of the imprinted genes Z16, Z19, and Z21 has a sensitivity of 94% and a specificity of 96% for determining breast tumors.

In a second aspect, the present disclosure provides the use of the grading model according to the first aspect in the detection and/or treatment of tumors.

In a third aspect, the present disclosure provides a method for detecting a tumor. The method includes: determining a type and/or a benign and malignant degree of the tumor in a test sample using the grading model according to the first aspect.

According to an embodiment of the present disclosure, the test sample is derived from a patient having or suspected of having a tumor selected from types of thyroid cancer, lung cancer, bladder cancer, prostate cancer, skin cancer, breast cancer, cervical cancer, intestinal cancer, stomach cancer, esophageal cancer, pancreatic cancer, and liver cancer.

In a fourth aspect, the present disclosure provides a method for monitoring a tumor. The method includes: determining, prior to and subsequent to medicament administration, a benign and malignant degree of the tumor in a patient using the grading model according to the first aspect, to monitor tumor development in the patient.

In the present disclosure, "the positive degree of only one of the imprinted genes Z16, Z19, and Z21 is low positive or above" means that the positive degree of any one of the imprinted genes Z16, Z19, and Z21 is low positive, moderate positive, or high positive, and the positive degree of the others of the imprinted genes Z16, Z19, and Z21 are negative or positive potential. For example, the positive degree of the imprinted gene Z16 is low positive, moderate positive, or high positive, and the positive degree of the imprinted genes Z19 and Z21 is negative or positive potential.

In the present disclosure, "positive potential or below" refers to negative or positive potential.

In the present disclosure, the imprinted gene Z21 is SNU13, also referred to as "NHP2L1", which are equivalent.

Additional aspects and advantages of the present disclosure will be set forth in part in the description, which follows and, in part, will be obvious from the description or may be learned by practice of the present disclosure.

## BRIEF DESCRIPTION OF THE DRAWINGS

The above and/or additional aspects and advantages of the present disclosure will become apparent and readily appreciated from the following description of the examples, taken in conjunction with the accompanying drawings, in which:

Fig. 1 shows four cell nuclei with different expression statuses of imprinted genes in a lung tissue section, where: *a* indicate a cell nucleus in which no marker is present and the imprinted genes are not expressed; b is a cell nucleus in which one imprinted gene marker is present; c is a cell nucleus in which two imprinted gene markers are present; and *d* is a cell nucleus in which more than two imprinted gene markers are present.

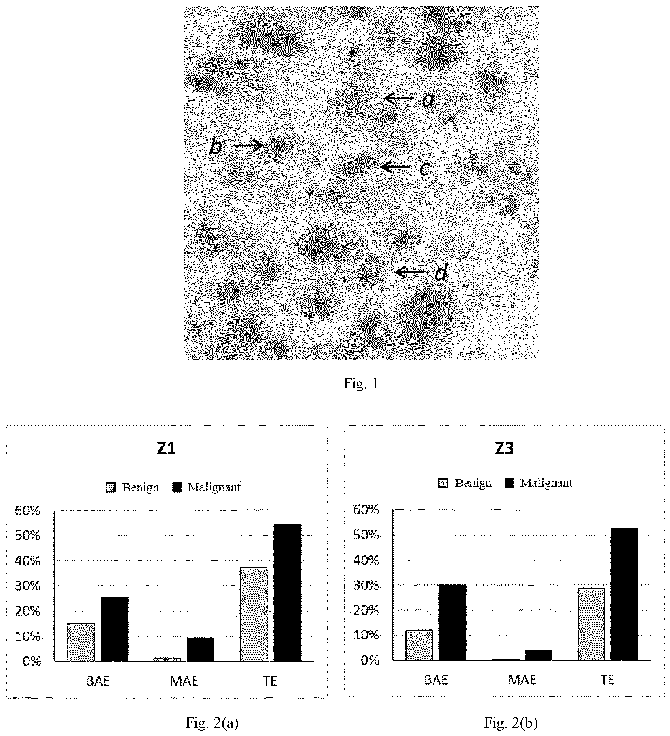

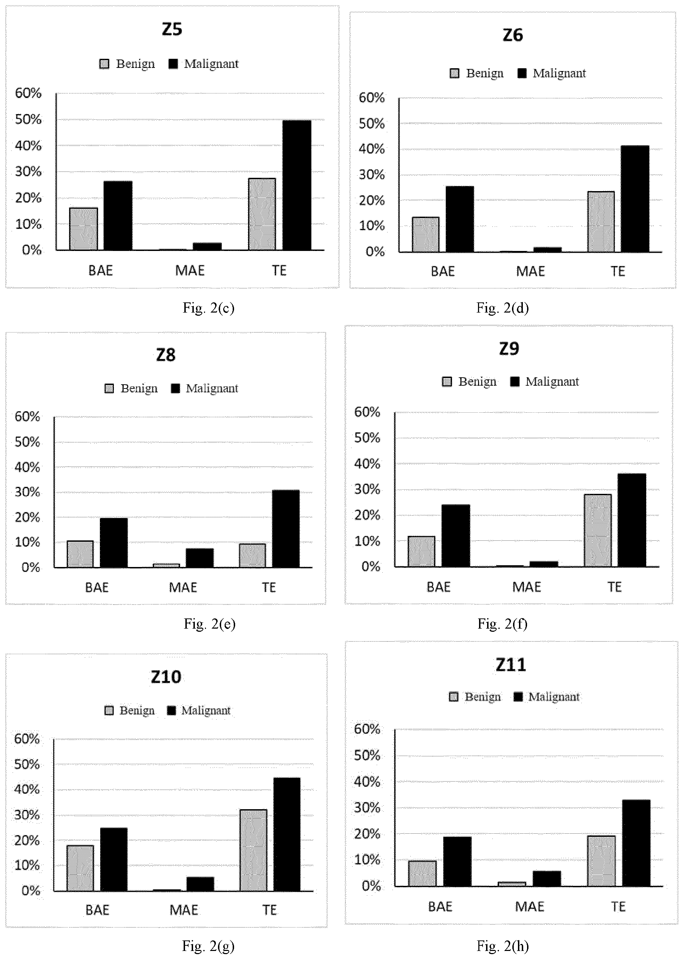

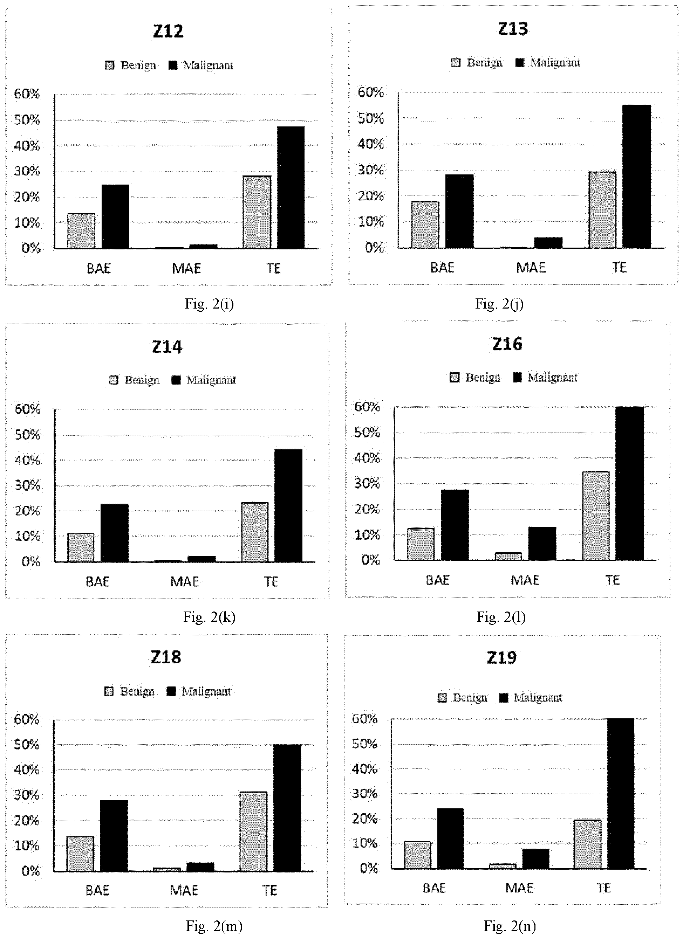

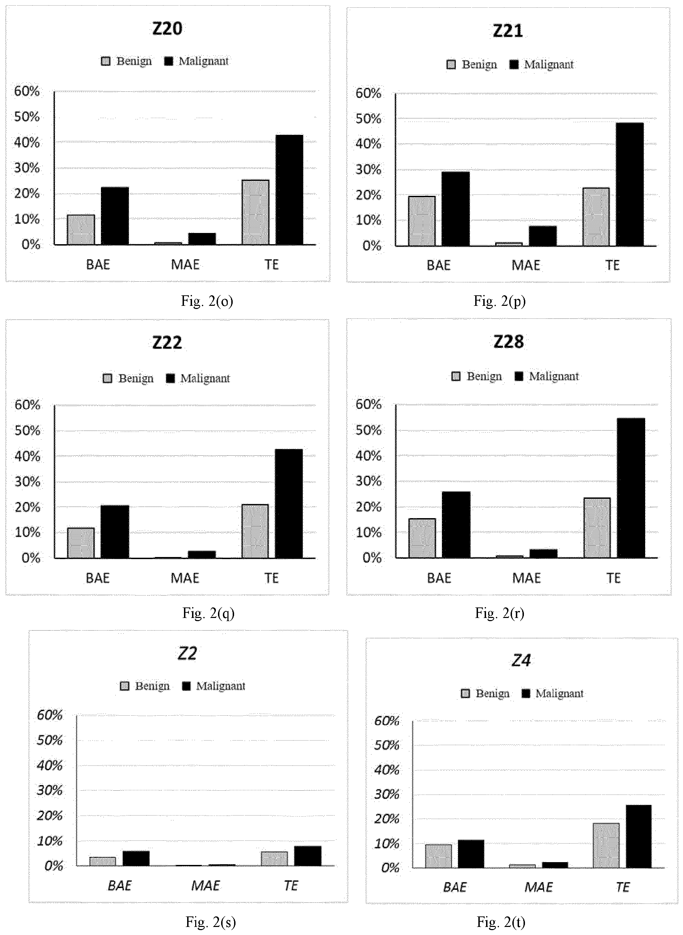

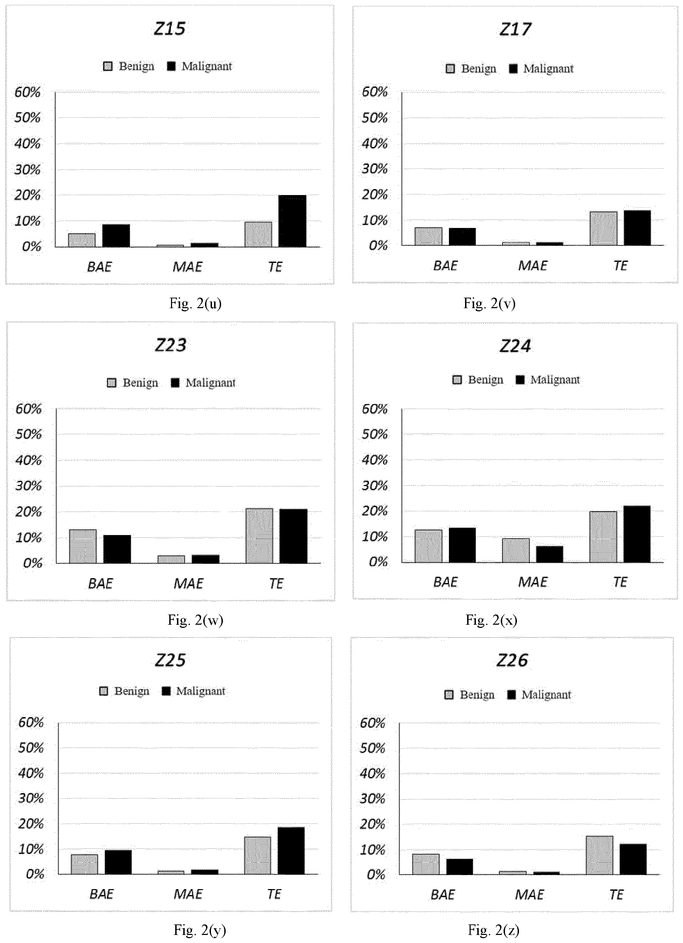

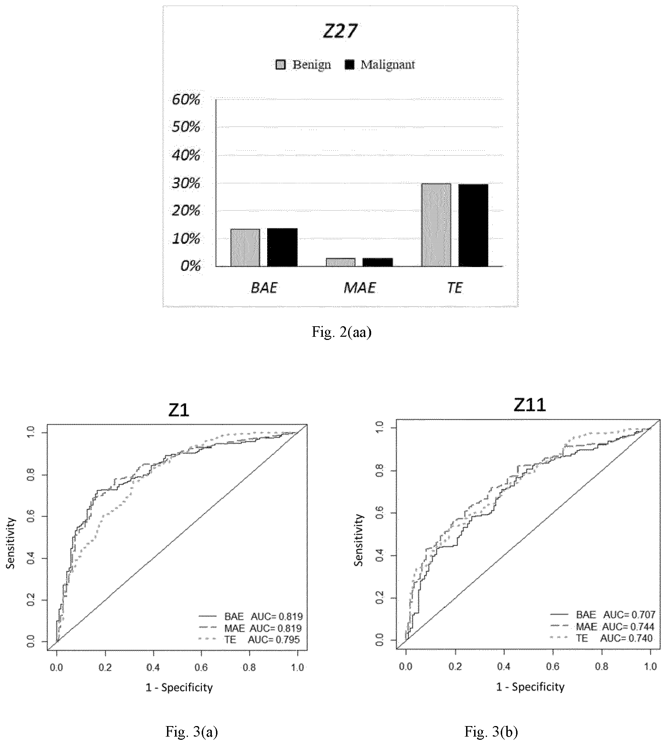

Fig. 2 shows expression statuses of BAE, MAE, and TE of imprinted genes Z1, Z3, Z5, Z6, Z8, Z9, Z10, Z11, Z12, Z13, Z14, Z16, Z18, Z19, Z20, Z21, Z22, and Z28 in benign and malignant lung tumors. Fig. 2(a) shows the expression statuses of BAE, MAE, and TE of the imprinted gene Z1 in benign and malignant lung tumors. Fig. 2(b) shows the expression statuses of BAE, MAE, and TE of the imprinted gene Z3 in benign and malignant lung tumors. Fig. 2(c) shows the expression statuses of BAE, MAE, and TE of the imprinted gene Z5 in benign and malignant lung tumors. Fig. 2(d) shows the expression statuses of BAE, MAE, and TE of the imprinted gene Z6 in benign and malignant lung tumors. Fig. 2(e) shows the expression statuses of BAE, MAE, and TE of the imprinted gene Z8 in benign and malignant lung tumors. Fig. 2(f) shows the expression status of BAE, MAE, and TE of the imprinted gene Z9 in benign and malignant lung tumors. Fig. 2(g) shows the expression status of BAE, MAE, and TE of the imprinted gene Z10 in benign and malignant lung tumors. Fig. 2(h) shows the expression statuses of BAE, MAE, and TE of the imprinted gene Z11 in benign and malignant lung tumors. Fig. 2(i) shows the expression statuses of BAE, MAE, and TE of the imprinted gene Z12 in benign and malignant lung tumors. Fig. 2(j) shows the expression statuses of BAE, MAE, and TE of the imprinted gene Z13 in benign and malignant lung tumors. Fig. 2(k) shows the expression statuses of BAE, MAE, and TE of the imprinted gene Z14 in benign and malignant lung tumors. Fig. 2(l) shows the expression statuses of BAE, MAE, and TE of the imprinted gene Z16 in benign and malignant lung tumors. Fig. 2(m) shows the expression statuses of BAE, MAE, and TE of the imprinted gene Z18 in benign and malignant lung tumors. Fig. 2(n) shows the expression statuses of BAE, MAE, and TE of the imprinted gene Z19 in benign and malignant lung tumors. Fig. 2(o) shows the expression statuses of BAE, MAE, and TE of imprinted gene Z20 in benign and malignant lung tumors. Fig. 2(p) shows the expression statuses of BAE, MAE, and TE of the imprinted gene Z21 in benign and malignant lung tumors. Fig. 2(q) shows the expression statuses of BAE, MAE, and TE of the imprinted gene Z22 in benign and malignant lung tumors. Fig. 2(r) shows the expression statuses of BAE, MAE, and TE of imprinted gene Z28 in benign and malignant lung tumors. Fig. 2(s) shows the expression statuses of BAE, MAE, and TE of the imprinted gene Z2 in benign and malignant lung tumors. Fig. 2(t) shows the expression statuses of BAE, MAE, and TE of the imprinted gene Z4 in benign and malignant lung tumors. Fig. 2(u) shows the expression statuses of BAE, MAE, and TE of the imprinted gene Z15 in benign and malignant lung tumors. Fig. 2(v) shows the expression statuses of BAE, MAE, and TE of the imprinted gene Z17 in benign and malignant lung tumors. Fig. 2(w) shows the expression statuses of BAE, MAE, and TE of the imprinted gene Z23 in benign and malignant lung tumors. Fig. 2(x) shows the expression statuses of BAE, MAE, and TE of the imprinted gene Z24 in benign and malignant lung tumors. Fig. 2(y) shows the expression statuses of BAE, MAE, and TE of the imprinted gene Z25 in benign and malignant lung tumors. Fig. 2(z) shows the expression statuses of BAE, MAE, and TE of the imprinted gene Z26 in benign and malignant lung tumors. Fig. 2(aa) shows the expression statuses of BAE, MAE, and TE of the imprinted gene Z27 in benign and malignant lung tumors.

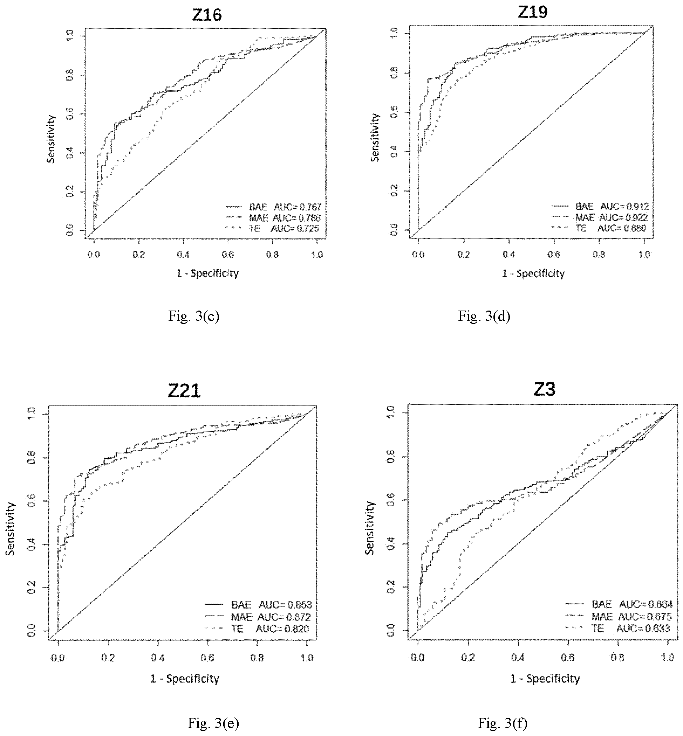

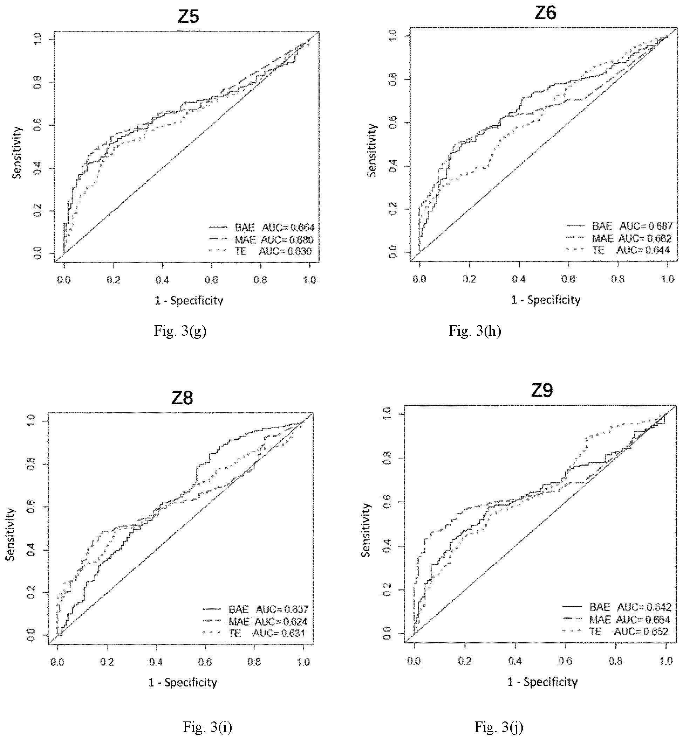

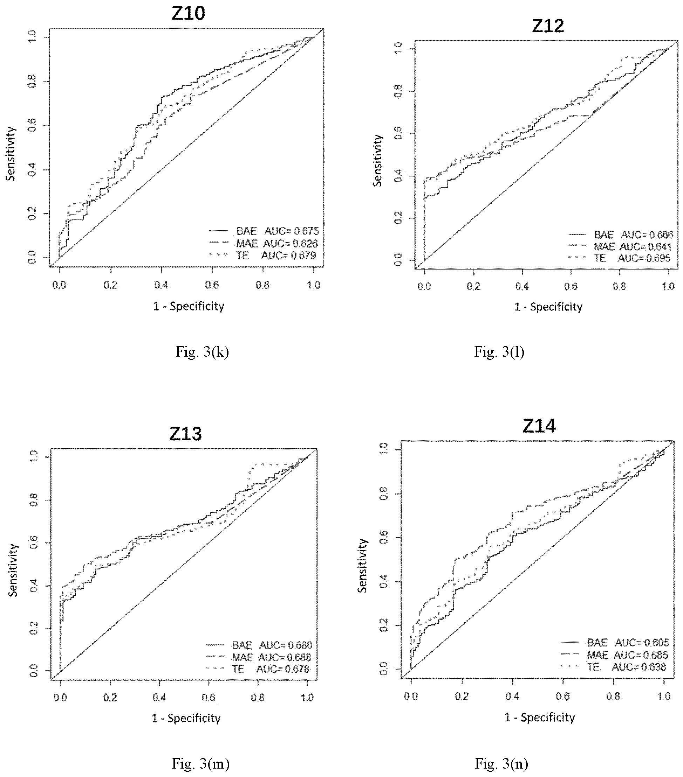

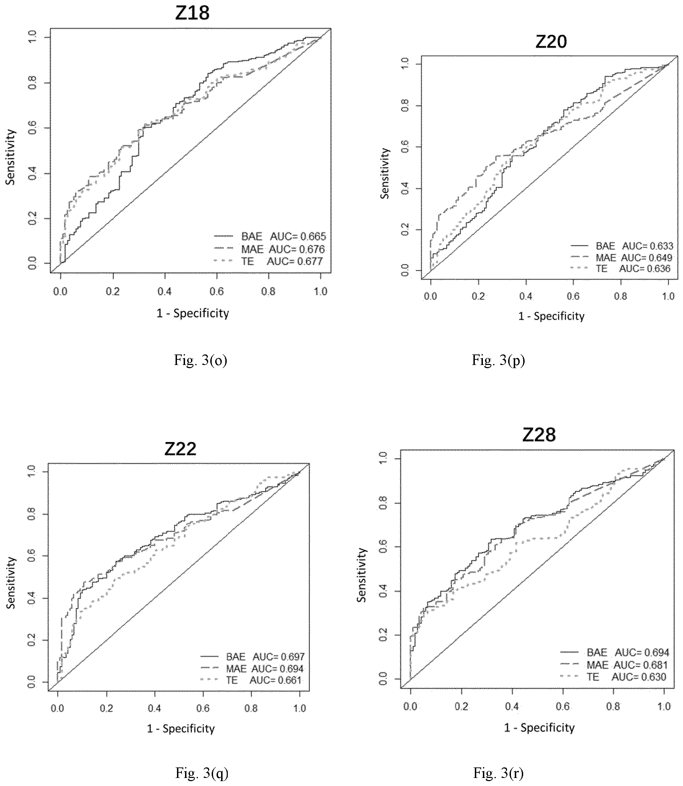

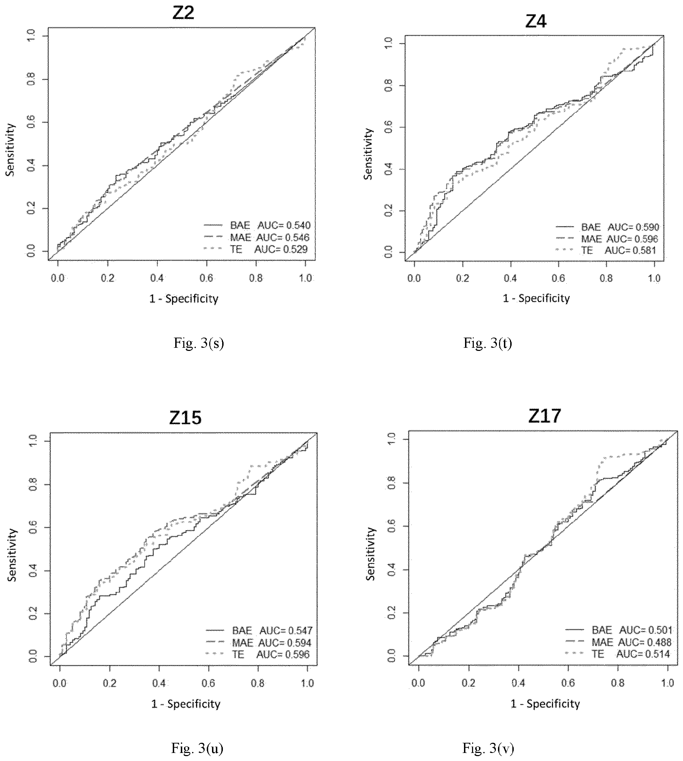

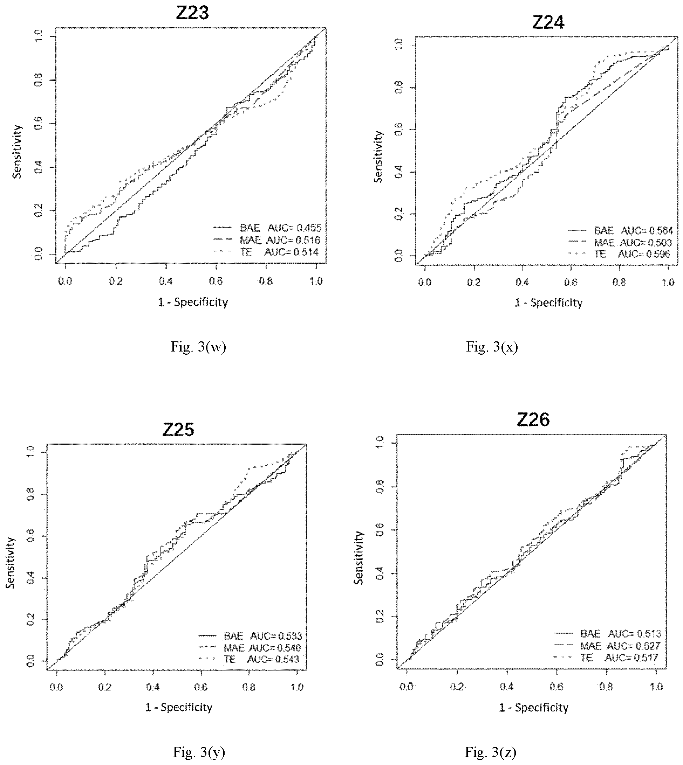

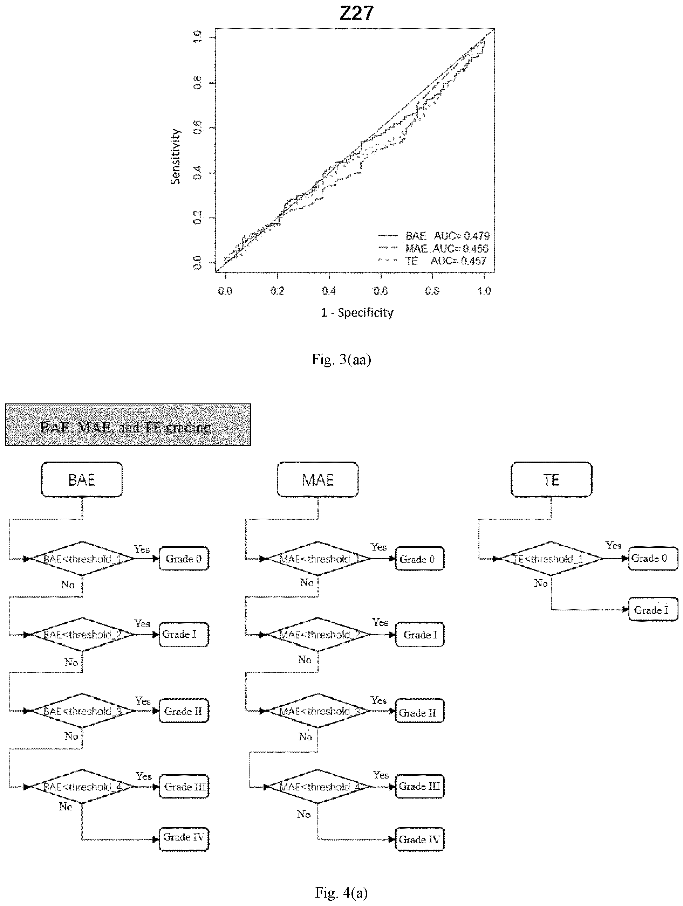

Fig. 3 shows ROC curves of imprinted genes in twelve tumors. Fig. 3(a) shows ROC curves of imprinted gene Z1. Fig. 3(b) shows ROC curves of imprinted gene Z11. Fig. 3(c) shows ROC curves of imprinted gene Z16. Fig. 3(d) shows ROC curves of imprinted gene Z19. Fig. 3(e) shows ROC curves of imprinted gene Z21. Fig. 3(f) shows ROC curves of imprinted gene Z3. Fig. 3(g) shows ROC curves of imprinted gene Z5. Fig. 3(h) shows ROC curves of imprinted gene Z6. Fig. 3(i) shows ROC curves of imprinted gene Z8. Fig. 3(j) shows ROC curves of imprinted gene Z9. Fig. 3(k) shows ROC curves of imprinted gene Z10. Fig. 3(l) shows ROC curves of imprinted gene Z12. Fig. 3(m) shows ROC curves of imprinted gene Z13. Fig. 3(n) shows ROC curves of imprinted gene Z14. Fig. 3(o) shows ROC curves of imprinted gene Z18. Fig. 3(p) shows ROC curves of imprinted gene Z20. Fig. 3(q) shows ROC curves of imprinted gene Z22. Fig. 3(r) shows ROC curves of imprinted gene Z28. Fig. 3(s) shows ROC curves of imprinted gene Z2. Fig. 3(t) shows ROC curves of imprinted gene Z4. Fig. 3(u) shows ROC curves of imprinted gene Z15. Fig. 3(v) shows ROC curves of imprinted gene Z17. Fig. 3(w) shows ROC curves of imprinted gene Z23. Fig. 3(x) shows ROC curves of imprinted gene Z24. Fig. 3(y) shows ROC curves of imprinted gene Z25. Fig. 3(z) shows ROC curves of imprinted gene Z26. Fig. 3(aa) shows ROC curves of imprinted gene Z27.

Fig. 4 is a schematic diagram of a grading method of imprinted genes. Fig. 4(a) shows a grading method for BAE, MAE, and TE of respective imprinted genes. Fig. 4(b) shows a grading method for a positive degree of an imprinted genes. Fig. 4(c) shows a method for predicting a benign and malignant degree of a tumor through a combination of multiple genes.

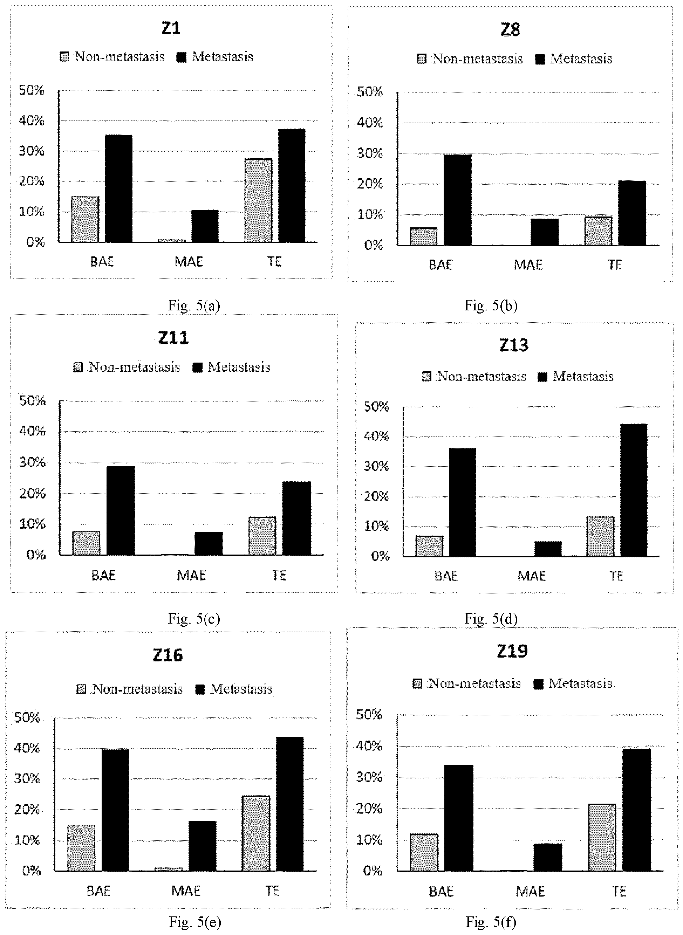

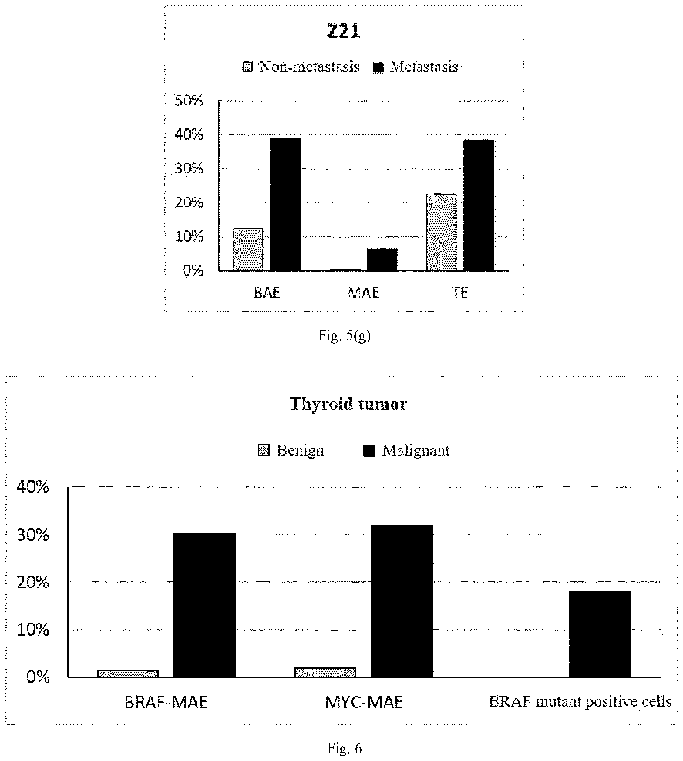

Fig. 5 shows expression statuses of BAE, MAE, and TE of imprinted genes Z1, Z8, Z11, Z13, Z16, Z19, and Z21 in breast cancer sentinel lymph node samples without and with lymph node metastasis. Fig. 5(a) shows the expression statuses of BAE, MAE, and TE of the imprinted gene Z1 in sentinel lymph node samples of breast cancer without and with lymph node metastasis. Fig. 5(b) shows the expression statuses of BAE, MAE, and TE of the imprinted gene Z8 in sentinel lymph node samples of breast cancer without and with lymph node metastasis. Fig. 5(c) shows the expression statuses of BAE, MAE, and TE of the imprinted gene Z11 in sentinel lymph node samples of breast cancer without and with lymph node metastasis. Fig. 5(d) shows the expression statuses of BAE, MAE, and TE of the imprinted gene Z13 in sentinel lymph node samples of breast cancer without and with lymph node metastasis. Fig. 5(e) shows the expression statuses of BAE, MAE, and TE of the imprinted gene Z16 in sentinel lymph node samples of breast cancer without and with lymph node metastasis. Fig. 5(f) shows the expression statuses of BAE, MAE, and TE of the imprinted gene Z19 in sentinel lymph node samples of breast cancer without and with lymph node metastasis. Fig. 5(g) shows the expression statuses of BAE, MAE, and TE of the imprinted gene Z21 in sentinel lymph node samples of breast cancer without and with lymph node metastasis.

Fig. 6 shows a multi-allelic expression of BRAF gene, a multi-allelic expression of MYC gene, and a proportion of BRAF gene mutant positive cells in benign and malignant thyroid tumors.

Fig. 7 shows statuses of multi-allelic expressions of CDKN2A, HER2, and MYC genes in benign and malignant lung tumors.

Fig. 8 shows a proportion of GRP and SYP gene-positive cells in non-small cell carcinoma of lung and small cell carcinoma of lung.

Fig. 9 shows statuses of multi-allelic expressions of CDKN2A and MYC genes in benign and malignant bladder tumors.

Fig. 10 shows statuses of multi-allelic expressions of HER2 and BRAF genes in benign and malignant breast tumors.

## DETAILED DESCRIPTION

The examples of the present disclosure will be described in detail below, which are exemplary and intended to be illustrative of the present disclosure, and should not be construed as limitations on the present disclosure.

In addition, the terms "first" and "second" are used only for the purpose of description and should not be construed as indicating or implying relative importance or implicitly indicating the number of the indicated technical features. Therefore, a feature defined by "first" or "second" may explicitly or implicitly include at least one such feature. In the description of the present disclosure, "plurality" refers to at least two, such as two or three, unless otherwise specifically defined.

According to an embodiment of the present disclosure, the expression status of imprinted gene is detected by *in situ* hybridization, including: designing a probe targeting an intron sequence of the imprinted gene, and hybridizing the probe with a nascent RNA expressed by the imprinted gene to display the number of alleles expressed by the imprinted gene in cell nucleus. Pre-RNA or nascent RNA containing exons and introns are first formed during gene transcription, and then the introns are cleaved and rapidly degraded. Thus, only the pre-RNA or nascent RNA, rather than mRNA containing only exons, can be detected using a probe targeting the intron. Since the intron cleavage of the pre-RNA or nascent RNA is performed at the same time as the transcription, the signal markers of the pre-RNA or nascent RNA actually display the spatial sites of the imprinted genes that are being transcribed in the cell nucleus, while the spatial sites of the imprinted genes that are not being transcribed are not detected as the pre-RNA or nascent RNA is not generated. By means of the RNA *in situ* hybridization, it can be detected that no marker or only one marker is present in the cell nucleus when the imprinted gene is not expressed in a normal case or only one allele is expressed, and it can be detected that two or more markers are present in the cell nucleus when abnormal expression statuses of two or more alleles of the imprinted genes occur in cancer. Therefore, based on a proportion of cells expressing imprinted genes in a tumor sample and a proportion of cells with an abnormal expression status of the imprinted genes, the benign and malignant degree of the tumor can be graded. Similarly, when a copy number abnormality of non-imprinted genes occurs in cancer, three or more markers can be detected by the specific RNA *in situ* hybridization of the present disclosure. A proportion of cells with an abnormal expression status of the non-imprinted genes in a tumor sample serve as an auxiliary to assist the imprinted genes to determine the benign and malignant degree of a tumor. The methods for detecting the expression statuses of imprinted and non-imprinted genes include, but are not limited to, those described above. Other available methods known in the related art also fall within the scope of the present disclosure.

According to an embodiment of the present disclosure, the *in situ* hybridization employs the RNAscope *in situ* hybridization.

According to an embodiment of the present disclosure, the RNAscope *in situ* hybridization method uses a single- or multi-channel coloring kit or a single- or multi-channel fluorescence kit, and preferably, a single-channel red/brown coloring kit or a multi-channel fluorescence kit.

According to an embodiment of the present disclosure, based on the number of alleles expressed by an imprinted gene in the cell nucleus, an expression status of the imprinted gene is divided into no expression of the imprinted gene, single-allelic expression of the imprinted gene, bi-allelic expression of the imprinted gene, and multi-allelic expression of the imprinted gene.

The "no expression of the imprinted gene" means that no imprinted gene marker is present in the cell nucleus of a sample.

The "single-allelic expression of the imprinted gene" means that one imprinted gene marker is present in the cell nucleus of the sample.

The "bi-allelic expression of the imprinted gene" means that two imprinted gene markers are present in the cell nucleus of the sample; and

The "multi-allelic expression of the imprinted gene" means that more than two imprinted gene markers are present in the cell nucleus of the sample.

According to an embodiment of the present disclosure, the total expression (TE), single-allelic expression (SAE), bi-allelic expression (BAE), and multi-allelic expression (MAE) are calculated based on the following formulas:

$$TE = (b+c+d)/(a+b+c+d);$$

$$SAE = b/(b+c+d);$$

$$BAE = c/(b+c+d);$$

$$MAE = d/(b+c+d);$$

where:

a represents cell nuclei in which no marker is present and the imprinted genes are not expressed after the cell is stained with hematoxylin; b represents cell nuclei in which one red/brown marker is present and only one allele of the imprinted gene is expressed after the cell is stained with hematoxylin; c represents cell nuclei in which two red/brown markers are present and two alleles of the imprinted gene are expressed after the cell is stained with hematoxylin; and d represents cell nuclei in which more than two red/brown markers are present and more than two alleles of the imprinted gene are expressed after the cell is stained with hematoxylin.

According to an embodiment of the present disclosure, the combination of imprinted genes Z16, Z19, and Z21 is used for detecting whether a tumor has lymphatic metastasis.

Based on the combination of the imprinted genes Z16, Z19, and Z21, the imprinted gene Z3 is additionally used for detecting early small cell carcinoma, including small cell carcinoma of lung, small cell carcinoma of bladder, and small cell carcinoma of cervix uteri.

According to an embodiment of the present disclosure, based on the combination of any one or more of imprinted genes Z1, Z3, Z5, Z6, Z8, Z9, Z10, Z11, Z12, Z13, Z14, Z18, Z20, Z22, and Z28 with the imprinted genes Z16, Z19, and Z21, non-imprinted genes BRAF and/or MYC can also additionally used for the diagnosis of thyroid cancer.

BRAF and MYC are detected by in situ hybridization, including designing probes targeting introns of BRAF and MYC genes and hybridizing the probes with nascent RNAs expressed by the genes to display the number of alleles expressed by the genes in the cell nucleus.

For the BRAF gene, a probe targeting the V600E mutation site can also be designed and hybridized with mRNA to display the mRNA expression of the mutant gene.

According to an embodiment of the present disclosure, based on the combination of any one or more of imprinted genes Z1, Z3, Z5, Z6, Z8, Z9, Z10, Z11, Z12, Z13, Z14, Z18, Z20, Z22, and Z28 with the imprinted genes Z16, Z19, and Z21, non-imprinted genes HER2 and/or MYC can be additionally used for the diagnosis of lung cancer.

HER2 and MYC are detected by in situ hybridization, including designing probes targeting introns of HER2 and MYC genes and hybridizing the probes with nascent RNAs expressed by the genes to display the number of alleles expressed by the genes in the cell nucleus.

According to an embodiment of the present disclosure, based on the combination of any one or more of imprinted genes Z1, Z3, Z5, Z6, Z8, Z9, Z10, Z11, Z12, Z13, Z14, Z18, Z20, Z22, and Z28 with the imprinted genes Z16, Z19, and Z21, non-imprinted genes GRP and/or SYP can be additionally used for the diagnosis of small cell carcinoma of lung.

GRP and SYP are detected by in situ hybridization, including designing probes targeting GRP and SYP mRNAs and hybridizing the probes with the mRNAs to display the mRNA expressions of the genes.

According to an embodiment of the present disclosure, based on the combination of any one or more of imprinted genes Z1, Z3, Z5, Z6, Z8, Z9, Z10, Z11, Z12, Z13, Z14, Z18, Z20, Z22, and Z28 with the imprinted genes Z16, Z19, and Z21, non-imprinted genes CDKN2A and MYC can be additionally used for the diagnosis of bladder cancer. CDKN2A and MYC are detected by in situ hybridization, including designing probes targeting introns of CDKN2A and MYC genes and hybridizing the probes with nascent RNAs expressed by the genes to display the number of alleles expressed by the genes in the cell nucleus.

According to an embodiment of the present disclosure, based on the combination of any one or more of imprinted genes Z1, Z3, Z5, Z6, Z8, Z9, Z10, Z11, Z12, Z13, Z14, Z18, Z20, Z22, and Z28 with the imprinted genes Z16, Z19, and Z21, non-imprinted genes HER2 and/or BRAF can be additionally used for the diagnosis of breast cancer.

HER2 and BRAF are detected by in situ hybridization, including designing probes targeting introns of HER2 and BRAF genes and hybridizing the probes with nascent RNAs expressed by the genes to display the number of alleles expressed by the genes in the cell nucleus.

The present disclosure will be described with reference to specific examples, which are merely for illustration rather than limiting the present disclosure in any way.

**Example 1: Analysis of expression statuses of imprinted genes in tissue sections of benign and malignant lung tumors**

(1) Ten cases of benign lung tumor tissues and 20 cases of malignant lung tumor tissues were obtained and fixed in 10% neutral formalin solution for 24 hours to prevent RNA degradation. The tissues were embedded in paraffin, cut into sections with a thickness of 10 μm, and adhered to glass slides of superfrost plus. The sections were baked in an oven at 40°C for more than 3 h.

(2) The samples were dewaxed by the sample treatment method of RNAscope, blocked for endogenous peroxidase activity, and enhanced permeability to expose RNA molecules.

(3) Probe design: specific probes were designed based on intron sequences of imprinted genes Z1 (GNAS), Z3 (PEG10), Z5 (MEST), Z6 (PLAGL1), Z8 (DCN), Z9 (DLK1), Z10 (GATM), Z11 (GRB10), Z12 (PEG3), Z13 (SGCE), Z14 (SLC38A4), Z16 (SNRPN/SNURF), Z18 (GATA3), Z19 (HM13), Z20 (KCNQ1), Z21 (SNU13), Z22 (PON2), Z28 (ZIC1), Z2 (IGF2), Z4 (IGF2R), Z15 (DIRAS3), Z17 (ALDH1L1), Z23 (RASGRF1), Z24 (RB1), Z25 (TFPI2), Z26 (TRAPPC9), and Z27 (UBE3A).

(4) The probes obtained in step (3) and the samples were subjected to RNAscope in situ hybridization using a kit.

(5) Signal amplification and hematoxylin staining were performed, and the expression of the imprinted genes was analyzed by microscopic imaging.

In this example, the expression of an imprinted gene includes no expression of the imprinted gene, single-allelic expression of the imprinted gene, bi-allelic expression of the imprinted gene, or multi-allelic expression of the imprinted gene. Among them, "no expression of the imprinted gene" means that no imprinted gene marker is present in cell nuclei of the sample; "single-allelic expression of the imprinted gene" means that one imprinted gene marker is present in the in cell nuclei of the sample; "bi-allelic expression of the imprinted gene" means that two imprinted gene markers are present in the in cell nuclei of the sample; and "multi-allelic expression of the imprinted gene" means that more than two imprinted gene markers are present in the in cell nuclei of the sample.

The results of *in situ* hybridization were observed under a microscope. As shown in Fig. 1, 1,200 to 2,000 cells were counted, and total expression (TE), single-allelic expression (SAE), bi-allelic expression (BAE), and multi-allelic expression (MAE) were calculated based on the following formulas:

$$TE = (b+c+d)/(a+b+c+d);$$

$$SAE = b/(b+c+d);$$

$$BAE = c/(b+c+d);$$

$$MAE = d/(b+c+d),$$

where:
a denotes the number of cells in which no marker is present in a cell nucleus and the imprinted genes are not expressed; b denotes the number of cells in which one imprinted gene marker is present in the cell nucleus; c denotes the number of cells in which two imprinted gene markers are present in the cell nucleus; and d denotes the number of cells in which more than two imprinted gene markers are present in the cell nucleus

The quantitative results are shown in Fig. 2, i.e., Fig. 2(a) to Fig. (aa). TE, BAE, and MAE of the imprinted genes Z1, Z3, Z5, Z6, Z8, Z9, Z10, Z11, Z12, Z13, Z14, Z16, Z18, Z19, Z20, Z21, Z22, and Z28 were significantly different in benign and malignant lung tumors, while TE, BAE, and MAE of the imprinted genes Z2, Z4, Z15, Z17, Z23, Z24, Z25, Z26, and Z27 were less different in benign and malignant lung tumors.

**Example 2: Analysis of expression statuses of 27 imprinted genes in twelve tumor tissue sections and establishment of grading model of benign and malignant degree**

10 cases of benign tumor tissues and 20 cases of malignant tumor tissues were obtained from each of thyroid, bladder, prostate, skin, breast, cervix, intestine, stomach, esophagus, pancreas, and liver. These tissues were fixed, embedded, cut into sections, and subjected to RNAscope *in situ* hybridization using the same method as described in Example 1.

BAE, MAE, and TE of imprinted genes were quantified using the method as described in Example 1. The quantitative data from eleven tumors in this example and data from lung tumor tissue samples in Example 1 were combined. ROC curves were made based on data of the BAE, MAE, and TE from 120 cases of benign tumor samples and 240 cases of malignant tumor samples for the respective genes, to analyze the ability of each gene to distinguish benign and malignant tumors. The results are shown in Fig. 3, i.e., Fig. 3(a) to Fig. (aa), and the area under the curve (AUC) of the BAE, MAE, and TE of each gene are shown in Table 1.

[Table 1]

| Imprinted gene | ROC AUC | | |
| --- | --- | --- | --- |
| | BAE | MAE | TE |
| Z1 | 0.819 | 0.819 | 0.795 |
| Z11 | 0.707 | 0.744 | 0.740 |

(continued)

| Imprinted gene | ROC AUC | | |
|---|---|---|---|
| | BAE | MAE | TE |
| Z16 | 0.767 | 0.786 | 0.725 |
| Z19 | 0.912 | 0.922 | 0.880 |
| Z21 | 0.853 | 0.872 | 0.820 |
| Z3 | 0.664 | 0.675 | 0.633 |
| Z5 | 0.664 | 0.680 | 0.630 |
| Z6 | 0.687 | 0.662 | 0.644 |
| Z8 | 0.637 | 0.624 | 0.631 |
| Z9 | 0.642 | 0.664 | 0.652 |
| Z10 | 0.675 | 0.626 | 0.679 |
| Z12 | 0.666 | 0.641 | 0.695 |
| Z13 | 0.680 | 0.688 | 0.678 |
| Z14 | 0.605 | 0.685 | 0.638 |
| Z18 | 0.665 | 0.676 | 0.677 |
| Z20 | 0.663 | 0.649 | 0.636 |
| Z22 | 0.697 | 0.694 | 0.661 |
| Z28 | 0.694 | 0.681 | 0.630 |
| Z2 | 0.540 | 0.546 | 0.529 |
| Z4 | 0.590 | 0.596 | 0.581 |
| Z15 | 0.547 | 0.594 | 0.596 |
| Z17 | 0.501 | 0.488 | 0.514 |
| Z23 | 0.455 | 0.516 | 0.514 |
| Z24 | 0.564 | 0.503 | 0.596 |
| Z25 | 0.533 | 0.540 | 0.543 |
| Z26 | 0.513 | 0.527 | 0.517 |
| Z27 | 0.479 | 0.456 | 0.457 |

The AUC of the BAE, MAE, and TE of five genes, i.e., Z1, Z11, Z16, Z19, and Z21, were all greater than 0.7, indicating that Z1, Z11, Z16, Z19, and Z21 have a better ability to distinguish benign and malignant tumors. The AUC of the BAE, MAE, and TE of thirteen genes, i.e., Z3, Z5, Z6, Z8, Z9, Z10, Z12, Z13, Z14, Z18, Z20, Z22, and Z28, were in the range from 0.6 to 0.7, indicating that these genes have a certain ability to distinguish benign and malignant tumors. The AUC of the BAE, MAE, and TE of nine genes, i.e., Z2, Z4, Z15, Z17, Z23, Z24, Z25, Z26, and Z27, were all lower than 0.6, indicating that these genes can hardly distinguish benign and malignant tumors.

Five genes, i.e., Z1, Z11, Z16, Z19, and Z21, having a better ability to distinguish benign and malignant tumors, were selected to construct a model for identifying a benign and malignant degree of a tumor. Four points on ROC curves were selected as thresholds, i.e., threshold_1 to threshold_4. BAE and MAE of each gene were divided into 5 grades. The specific grading method was as follows:

Grade 0: BAE or MAE was less than threshold_1;
Grade I: BAE or MAE was greater than or equal to threshold_1 and less than threshold _2;
Grade II: BAE or MAE was greater than or equal to threshold_2 and less than threshold_3;
Grade III: BAE or MAE was greater than or equal to threshold_3 and less than threshold _4; and
Grade IV: BAE or MAE was greater than or equal to threshold_4.

TE of each gene was divided into two grades: Grad 0 and Grade I:

Grade 0: TE was less than threshold_1; and
Grade I: TE was greater than or equal to threshold_1.

The thresholds of the five genes, i.e., Z1, Z11, Z16, Z19, and Z21, were specifically selected in the following manner:

for BAE of the five genes, i.e., Z1, Z11, Z16, Z19, and Z21, the thresholds corresponding to a sensitivity of 90%, 60%, 30%, and 10% on ROC curves were selected as threshold_1, threshold_2, threshold_3, and threshold_4, respectively;
for MAE of the five genes, i.e., Z1, Z11, Z16, Z19, and Z21, the thresholds corresponding to a sensitivity of 95%, 80%, 70%, and 50% on ROC curves were selected as threshold_1, threshold_2, threshold_3, and threshold_4, respectively; and
for TE of the five genes, i.e., Z1, Z11, Z16, Z19, and Z21, the threshold corresponding to a sensitivity of 99% on ROC curves was selected as threshold_1.

The thresholds of the five genes, i.e., Z1, Z11, Z16, Z19, and Z21, are shown in Table 2.

[Table 2]

| Imprinted gene | Parameters | Threshold_1 | Threshold_2 | Threshold_3 | Threshold_4 |
|---|---|---|---|---|---|
| Z1 | BAE | 10% | 18% | 25% | 30% |
| | MAE | 1.3% | 3.3% | 4.7% | 7.8% |
| | TE | 10% | | | |
| Z11 | BAE | 8% | 16% | 23% | 28% |
| | MAE | 0.3% | 2.2% | 3.1% | 6.1% |
| | TE | 6% | | | |
| Z16 | BAE | 15% | 20% | 26% | 30% |
| | MAE | 1.4% | 3.4% | 5.5% | 9.0% |
| | TE | 17% | | | |
| Z19 | BAE | 12% | 20% | 26% | 30% |
| | MAE | 1.4% | 3.4% | 6.3% | 9.0% |
| | TE | 11% | | | |
| Z21 | BAE | 12% | 16% | 20% | 25% |
| | MAE | 1.3% | 3.2% | 4.9% | 6.2% |
| | TE | 14% | | | |

Combining the grades of BAE, MAE, and TE of each gene, the positive degree of each gene was divided into 5 grades: negative, positive potential, low positive, moderate positive, and high positive. The specific grading method was as follows:

when TE of the imprinted gene was Grade 0, or when the TE of the imprinted gene was Grade I and BAE and MAE were both Grade 0, the positive degree of the imprinted gene was determined to be negative;
when TE of the imprinted gene was Grade I as well as at least one of BAE and MAE was Grade I and both were not Grade II, the positive degree of the imprinted gene was determined to be positive potential;
when TE of the imprinted gene was Grade I as well as at least one of BAE and MAE was Grade II and both were not Grade III, the positive degree of the imprinted gene was determined to be low positive;
when TE of the imprinted gene was Grade I as well as at least one of BAE and MAE was Grade III and both were not Grade IV, the positive degree of the imprinted gene was determined to be moderate positive; and
when TE of the imprinted gene was Grade I as well as at least one of BAE and MAE was Grade IV, the positive degree of the imprinted gene was determined to be high positive.

Two or more genes were arbitrarily selected from the five genes, Z1, Z11, Z16, Z19, and Z21, for combination. A tumor was divided into five grades based on the positive degree of these different genes: benign tumor, cancer potential, early cancer, intermediate cancer, and terminal cancer. The specific combination method was as follows:

when the positive degree of all the imprinted genes in the combination were negative, or when the positive degree of no more than one imprinted gene in the combination was positive potential, the benign and malignant degree of the tumor was determined to be benign tumor;
when the positive degree of at least two imprinted genes in the combination was positive potential, or when the positive degree of no more than one imprinted gene was low positive, the benign and malignant degree of the tumor was determined to be cancer potential;
when the positive degree of at least two imprinted genes in the combination was low positive, or when the positive degree of no more than one imprinted gene was moderate positive, the benign and malignant degree of the tumor was determined to be early cancer;
when the positive degree of at least two imprinted genes in the combination was moderate positive, or when the positive degree of no more than one imprinted gene was high positive, the benign and malignant degree of the tumor was determined to be intermediate cancer; and
when the positive degree of at least two imprinted genes in the combination was high positive, the benign and malignant degree of the tumor was determined to be terminal cancer.

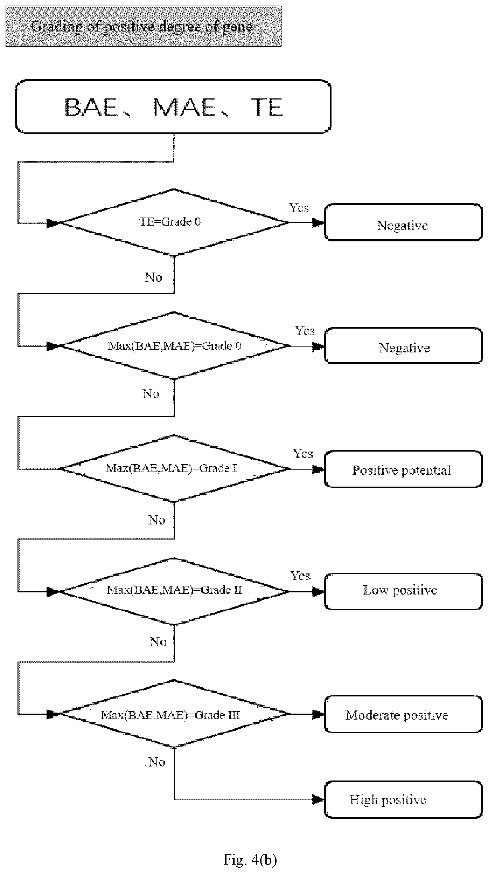

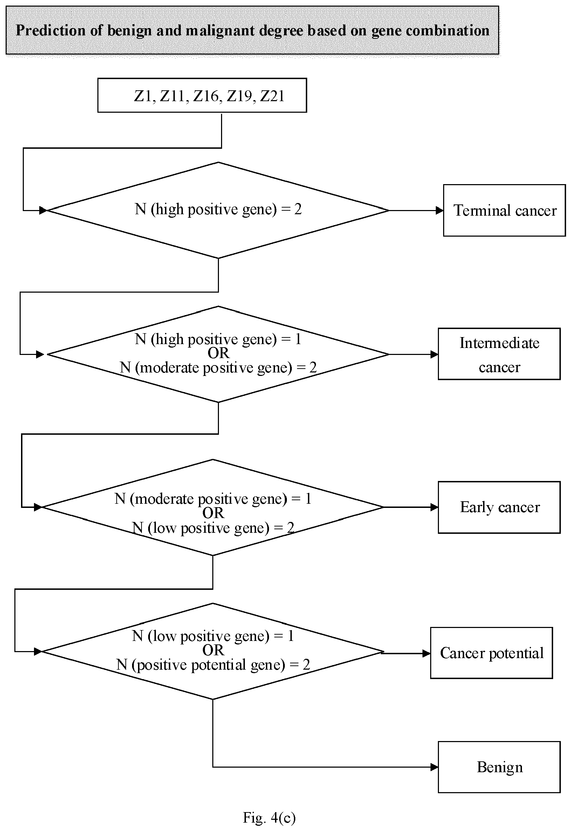

The grading method of BAE, MAE, and TE of a gene, the grading method of positive degree of a gene, and the method for gene combination are as shown in Fig. 4, i.e., Fig. 4(a) to Fig. 4(c).

Two or more genes were arbitrarily selected from the five genes, i.e., Z1, Z11, Z16, Z19, and Z21, for combination. Based on the above model, 120 cases of benign tumor samples and 240 cases of malignant tumor samples were divided into five grades, in which benign tumor and cancer potential were predicted to be benign, while early cancer, intermediate cancer, and terminal cancer were predicted to be malignant. The sensitivity and specificity of single gene and different gene combinations were evaluated. The results are shown in Table 3.

[Table 3]

| Gene or gene combination | Sensitivity | Specificity |
|---|---|---|
| Z1 | 80.4% | 70.8% |
| Z11 | 81.7% | 60.8% |
| Z16 | 82.1% | 80.0% |
| Z19 | 88.3% | 90.0% |
| Z21 | 82.9% | 74.2% |
| Z1+Z11 | 92.1% | 63.3% |
| Z1+Z16 | 93.8% | 75.8% |
| Z1+Z19 | 92.5% | 83.3% |
| Z1+Z21 | 92.5% | 76.7% |
| Z11+Z16 | 92.9% | 65.0% |
| Z11+Z19 | 90.8% | 68.3% |
| Z11+Z21 | 90.4% | 67.5% |
| Z16+Z19 | 94.6% | 90.0% |
| Z16+Z21 | 94.6% | 90.0% |
| Z19+Z21 | 88.3% | 92.5% |
| Z1+Z11+Z16 | 98.3% | 59.2% |
| Z1+Z11+Z19 | 98.8% | 61.7% |
| Z1+Z11+Z21 | 97.5% | 59.2% |

(continued)

| Gene or gene combination | Sensitivity | Specificity |
|---|---|---|
| Z1+Z16+Z19 | 98.8% | 75.8% |
| Z1+Z16+Z21 | 98.8% | 73.3% |
| Z1+Z19+Z21 | 96.3% | 75.8% |
| Z11+Z16+Z19 | 98.8% | 64.2% |
| Z11+Z16+Z21 | 98.3% | 64.2% |
| Z11+Z19+Z21 | 95.0% | 66.7% |
| Z16+Z19+Z21 | 98.3% | 90.0% |
| Z1+Z11+Z16+Z19 | 100.0% | 58.3% |
| Z1+Z11+Z16+Z21 | 99.2% | 56.7% |
| Z1+Z11+Z19+Z21 | 99.2% | 58.3% |
| Z1+Z16+Z19+Z21 | 99.6% | 73.3% |
| Z11+Z16+Z19+Z21 | 99.6% | 63.3% |
| Z1+Z11+Z16+Z19+Z21 | 100.0% | 55.8% |

As revealed in Table 3:

when only one of five genes, i.e., Z1, Z11, Z16, Z19, and Z21, was used, the sensitivity was not higher than 90%; when two genes were combined, the sensitivity was increased, with the combinations of Z16 and Z19, Z16 and Z21 having the highest sensitivity of 94.6%, and the combination of Z19 and Z21 having the highest specificity of 92.5%; when three genes were combined, the sensitivity was further increased, with the combinations of Z1+Z11+Z19, Z1+Z16+Z19, Z1+Z16+Z21, and Z11+Z16+Z19 having the highest sensitivity of 98.8%, but having a lower specificity only ranging from 61.7% to 75.8%, and the combination of Z16+Z19+Z21 having a slightly lower sensitivity of 98.3% and the highest specificity of 90.0%; and
when four or five genes were combined, the specificity was significantly reduced, all below 75%.

Therefore, taking both the sensitivity and specificity into consideration, the combination of three genes, i.e., Z16, Z19, and Z21, can achieve the optimal effect of distinguishing benign and malignant tumors.
In addition to these five genes, i.e., Z1, Z11, Z16, Z19, and Z21, ROC curves showed that thirteen genes, i.e., Z3, Z5, Z6, Z8, Z9, Z10, Z12, Z13, Z14, Z18, Z20, Z22, and Z28, also had a certain ability to distinguish benign and malignant tumors. In addition to the five genes, i.e., Z1, Z11, Z16, Z19, and Z21, by adding one or more of the above thirteen genes, the sensitivity can be further improved to avoid a missed diagnosis. In addition, when adjusting a grading model of Z1 and Z11 to appropriately improve the specificity, as an auxiliary to the combination of the genes Z16, Z19, and Z21, it can also play a role in improving the sensitivity.
One point on ROC curves of Z1, Z3, Z5, Z6, Z8, Z9, Z10, Z11, Z12, Z13, Z14, Z18, Z20, Z22, and Z28 was select as a threshold, threshold_1. The BAE, MAE, and TE of the fifteen genes were each divided into two grades. The specific grading method was as follows:

Grade 0: BAE, MAE, or TE was less than threshold_1; and
Grade I: BAE, MAE, or TE was greater than or equal to threshold_1.

Combining the grades of the BAE, MAE, and TE of each of the genes Z1, Z3, Z5, Z6, Z8, Z9, Z10, Z11, Z12, Z13, Z14, Z18, Z20, Z22, and Z28, the positive degree of each gene was divided into two grades: negative and positive. The specific grading method was as follows: when at least one of the TE, BAE, or MAE of the imprinted gene was Grade 0, the positive degree of the imprinted gene was determined to be negative; and when TE, BAE, and MAE of the imprinted gene were all Grade I, the positive degree of the imprinted gene was determined to be positive.
Since the combination of three genes, i.e., Z16, Z19, and Z21, had achieved very high sensitivity, in order to avoid a decrease of specificity due to the excessive increase of sensitivity, the threshold corresponding to a specificity of 95% on the ROC curves of BAE and MAE of each gene was selected as threshold_1, and the threshold corresponding to a sensitivity of 90% on the ROC curve of TE of each gene was selected as threshold_1.

The thresholds of BAE, MAE, and TE of each of the genes Z3, Z5, Z6, Z8, Z9, Z10, Z12, Z13, Z14, Z18, Z20, Z22, and Z28 are shown in Table 4.

[Table 4]

| Imprinted gene | BAE | MAE | TE |
|---|---|---|---|
| Z1 | 25% | 11.6% | 17% |
| Z3 | 13% | 6.0% | 5% |
| Z5 | 19% | 4.7% | 5% |
| Z6 | 19% | 5.6% | 7% |
| Z8 | 28% | 19.8% | 6% |
| Z9 | 13% | 7.2% | 5% |
| Z10 | 23% | 16.8% | 6% |
| Z11 | 25% | 11.8% | 12% |
| Z12 | 15% | 3.1% | 6% |
| Z13 | 16% | 7.4% | 7% |
| Z14 | 22% | 6.1% | 6% |
| Z18 | 26% | 18.6% | 8% |
| Z20 | 29% | 21.7% | 10% |
| Z22 | 21% | 14.0% | 6% |
| Z28 | 17% | 14.1% | 5% |

Based on the combination of three genes, i.e., Z16, Z19, and Z21, by combining the positive degree of Z3, Z5, Z6, Z8, Z9, Z10, Z12, Z13, Z14, Z18, Z20, Z22, and Z28, the benign and malignant degree of the tumor was further divided into two grades. The specific grading method was as follows:

when the positive degree of the imprinted genes Z16, Z19, and Z21 were all negative or positive potential, or when the positive degree of no more than one of the imprinted genes Z16, Z19, and Z21 was low positive and the positive degree of Z1, Z3, Z5, Z6, Z8, Z9, Z10, Z11, Z12, Z13, Z14, Z18, Z20, Z22, and Z28 were all negative, the benign and malignant degree of the tumor was determined to be benign tumor; and

when the positive degree of no more than one of the imprinted genes Z16, Z19, and Z21 was low positive and the positive degree of at least one of the imprinted genes Z1, Z3, Z5, Z6, Z8, Z9, Z10, Z11, Z12, Z13, Z14, Z18, Z20, Z22, and Z28 was positive, or when the positive degree of at least two of the imprinted genes Z16, Z19, and Z21 was low positive, or when the positive degree of at least one of the imprinted genes Z16, Z19, and Z21 was moderate positive, the benign and malignant degree of the tumor was determined to be malignant tumor.

The combination of the three genes, Z16, Z19, and Z21, was further combined with any one of the genes Z1, Z3, Z5, Z6, Z8, Z9, Z10, Z11, Z12, Z13, Z14, Z18, Z20, Z22, and Z28. The sensitivity and specificity for tumor diagnosis are shown in Table 5.

[Table 5]

| Combination of imprinted genes | Sensitivity | Specificity |
|---|---|---|
| Z16+Z19+Z21 | 98.3% | 90.0% |
| Z16+Z19+Z21+Z1 | 100.0% | 86.7% |
| Z16+Z19+Z21+Z3 | 99.2% | 89.2% |
| Z16+Z19+Z21+Z5 | 99.2% | 88.3% |
| Z16+Z19+Z21+Z6 | 98.8% | 90.0% |

(continued)

| Combination of imprinted genes | Sensitivity | Specificity |
|---|---|---|
| Z16+Z19+Z21+Z8 | 98.8% | 90.0% |
| Z16+Z19+Z21+Z9 | 100.0% | 86.7% |
| Z16+Z19+Z21+Z10 | 99.2% | 88.3% |
| Z16+Z19+Z21+Z11 | 100.0% | 86.7% |
| Z16+Z19+Z21+Z12 | 98.8% | 89.2% |
| Z16+Z19+Z21+Z13 | 99.6% | 90.0% |
| Z16+Z19+Z21+Z14 | 99.2% | 86.7% |
| Z16+Z19+Z21+Z18 | 98.8% | 87.5% |
| Z16+Z19+Z21+Z20 | 98.8% | 88.3% |
| Z16+Z19+Z21+Z22 | 98.8% | 87.5% |
| Z16+Z19+Z21+Z28 | 99.2% | 87.5% |

Table 5 reveals that each of the imprinted genes Z1, Z3, Z5, Z6, Z8, Z9, Z10, Z11, Z12, Z13, Z14, Z18, Z20, Z22, and Z28 can increase the sensitivity for tumor diagnosis, while having insignificant effect on the specificity.

It can be seen from ROC curves that the imprinted genes Z2, Z4, Z15, Z17, Z23, Z24, Z25, Z26, and Z27 almost had no ability to distinguish the benign and malignant degree of a tumor. Therefore, these genes are not used in the model.

**Example 3: Verification of identification model of benign and malignant degree based on imprinted genes in fine needle aspiration biopsy samples of benign and malignant thyroid tumors**

100 cases of fine needle aspiration samples of each of benign and malignant thyroid tumors were obtained, fixed in 10% neutral formalin solution for 24 hours, and then adhered to positively charged glass slides. The remaining steps were the same as those in Example 1.

The benign and malignant degree of the tumor was graded using the model constructed in Example 2 based on bi-allelic expression, multi-allelic expression, and total expression of imprinted genes in the samples.

A bi-allelic expression of the imprinted gene Z16 was divided into the following five different grades:

Grade 0: the bi-allelic expression of the imprinted gene Z16 was less than 15%;
Grade I: the bi-allelic expression of the imprinted gene Z16 was 15-20%;
Grade II: the bi-allelic expression of the imprinted gene Z16 was 20-26%;
Grade III: the bi-allelic expression of the imprinted gene Z16 was 26-30%; and
Grade IV: the bi-allelic expression of the imprinted gene Z16 was greater than 30%.

A multi-allelic expression of the imprinted gene Z16 was divided into the following five different grades:

Grade 0: the multi-allelic expression of the imprinted gene Z16 was less than 1.4%;
Grade I: the multi-allelic expression of the imprinted gene Z16 was 1.4-3.4%;
Grade II: the multi-allelic expression of the imprinted gene Z16 was 3.4-5.5%;
Grade III: the multi-allelic expression of the imprinted gene Z16 was 5.5-9%; and
Grade IV: the multi-allelic expression of the imprinted gene Z16 was greater than 9%.

A total expression of the imprinted gene Z16 was divided into the following two different grades:

Grade 0: the total expression of the imprinted gene Z16 was less than 17%; and
Grade I: the total expression of the imprinted gene Z16 was greater than or equal to 17%.

According to an embodiment of the present disclosure, a bi-allelic expression of the imprinted gene Z19 was divided into the following five different grades:

Grade 0: the bi-allelic expression of the imprinted gene Z19 was less than 12%;
Grade I: the bi-allelic expression of the imprinted gene Z19 was 12-20%;
Grade II: the bi-allelic expression of the imprinted gene Z19 was 20-26%;
Grade III: the bi-allelic expression of the imprinted gene Z19 was 26-30%; and
Grade IV: the bi-allelic expression of the imprinted gene Z19 was greater than 30%.

A multi-allelic expression of the imprinted gene Z19 was divided into the following five different grades:

Grade 0: the multi-allelic expression of the imprinted gene Z19 was less than 1.4%;
Grade I: the multi-allelic expression of the imprinted gene Z19 was 1.4-3.4%;
Grade II: the multi-allelic expression of the imprinted gene Z19 was 3.4-6.3%;
Grade III: the multi-allelic expression of the imprinted gene Z19 was 6.3-9%; and
Grade IV: the multi-allelic expression of the imprinted gene Z19 was greater than 9%.

A total expression of the imprinted gene Z19 was divided into the following two different grades:

Grade 0: the total expression of the imprinted gene Z19 was less than 11%; and
Grade I: the total expression of the imprinted gene Z19 was greater than or equal to 11%.

According to an embodiment of the present disclosure, a bi-allelic expression of the imprinted gene Z21 was divided into five different grades:

Grade 0: the bi-allelic expression of the imprinted gene Z21 was less than 12%;
Grade I: the bi-allelic expression of the imprinted gene Z21 was 12-16%;
Grade II: the bi-allelic expression of the imprinted gene Z21 was 16-20%;
Grade III: the bi-allelic expression of the imprinted gene Z21 was 20-25%; and
Grade IV: the bi-allelic expression of the imprinted gene Z21 was greater than 25%.

A multi-allelic expression of the imprinted gene Z21 was divided into the following five different grades:

Grade 0: the multi-allelic expression of the imprinted gene Z21 was less than 1.3%;
Grade I: the multi-allelic expression of the imprinted gene Z21 was 1.3-3.2%;
Grade II: the multi-allelic expression of the imprinted gene Z21 was 3.4-4.9%;
Grade III: the multi-allelic expression of the imprinted gene Z21 was 4.9-6.2%; and
Grade IV: the multi-allelic expression of the imprinted gene Z21 was greater than 6.2%.

A total expression of the imprinted gene Z21 was divided into the following two different grades:

Grade 0: the total expression of the imprinted gene Z21 was less than 14%; and
Grade I: the total expression of the imprinted gene Z21 was greater than or equal to 14%.

The positive degree of the imprinted genes Z16, Z19, and Z21 was divided into negative, positive potential, low positive, moderate positive, and high positive.
When the total expression of the imprinted gene was Grade 0, or when total expression of the imprinted gene was Grade I and the bi-allelic expression and multi-allelic expression were both Grade 0, the positive degree of the imprinted gene was determined to be negative.
When the total expression of the imprinted gene was Grade I as well as at least one of the bi-allelic expression and multi-allelic expression was Grade I and both were not Grade II, the positive degree of the imprinted gene was determined to be positive potential.
When the total expression of the imprinted gene was Grade I as well as at least one of the bi-allelic expression and multi-allelic expression was Grade II and both were not Grade III, the positive degree of the imprinted gene was determined to be low positive.
When the total expression of the imprinted gene was Grade I as well as at least one of the bi-allelic expression and multi-allelic expression was Grade III and both were not Grade IV, the positive degree of the imprinted gene was determined to be moderate positive.
When the total expression of the imprinted gene was Grade I as well as at least one of the bi-allelic expression and multi-allelic expression was Grade IV, the positive degree of the imprinted gene was determined to be high positive.
The benign and malignant degree of the tumor was divided into benign tumor, cancer potential, early cancer, intermediate cancer, and terminal cancer.

When the positive degree of the imprinted genes Z16, Z19, and Z21 were all negative, or when the positive degree of no more than one of the imprinted genes Z16, Z19, and Z21 was positive potential, the benign and malignant degree of the tumor was determined to be benign tumor.

When the positive degree of at least two of the imprinted genes Z16, Z19, and Z21 were positive potential, or the positive degree of no more than one of the imprinted genes Z16, Z19, and Z21 was low positive, the benign and malignant degree of the tumor was determined to be cancer potential.

When the positive degree of at least two of the imprinted genes Z16, Z19, and Z21 was low positive, or the positive degree of no more than one of the imprinted genes Z16, Z19, and Z21 was moderate positive, the benign and malignant degree of the tumor was determined to be early cancer.

When the positive degree of at least two of the imprinted genes Z16, Z19, and Z21 was moderate positive, or when the positive degree of no more than one of the imprinted genes Z16, Z19, and Z21 was high positive, the benign and malignant degree of the tumor was determined to be intermediate cancer.

When the positive degree of at least two of the imprinted genes Z16, Z19, and Z21 was high positive, the benign and malignant degree of the tumor was determined to be terminal cancer.

The samples determined as benign tumor and cancer potential based on the benign and malignant degree of the tumor were recorded as benign samples, and the samples determined as early cancer, intermediate cancer, and terminal cancer based on the benign and malignant degree of the tumor were recorded as malignant samples. The results were compared with the benign and malignant degree of the samples pathologically diagnosed after surgery, to evaluate the sensitivity and specificity of the model.

Table 6 shows the sensitivity and specificity of models of the combination of imprinted genes Z16, Z19, and Z21 as well as the combination of imprinted gene Z1, Z3, Z5, Z6, Z8, Z9, Z10, Z11, Z12, Z13, Z14, Z18, Z20, Z22, or Z28 with the imprinted genes Z16, Z19, and Z21, for detecting the benign and malignant degree of thyroid tumor.

[Table 6]

| Combination of imprinted genes | Sensitivity | Specificity |
|---|---|---|
| Z16+Z19+Z21 | 96% | 91% |
| Z16+Z19+Z21+Z1 | 100% | 82% |
| Z16+Z19+Z21+Z3 | 98% | 87% |
| Z16+Z19+Z21+Z5 | 97% | 89% |
| Z16+Z19+Z21+Z6 | 98% | 89% |
| Z16+Z19+Z21+Z8 | 97% | 91% |
| Z16+Z19+Z21+Z9 | 97% | 90% |
| Z16+Z19+Z21+Z10 | 98% | 89% |
| Z16+Z19+Z21+Z11 | 100% | 88% |
| Z16+Z19+Z21+Z12 | 97% | 91% |
| Z16+Z19+Z21+Z13 | 99% | 90% |
| Z16+Z19+Z21+Z14 | 97% | 91% |
| Z16+Z19+Z21+Z18 | 99% | 89% |
| Z16+Z19+Z21+Z20 | 98% | 87% |
| Z16+Z19+Z21+Z22 | 97% | 89% |
| Z16+Z19+Z21+Z28 | 100% | 85% |

As shown in Table 6, when only three imprinted genes, i.e., Z16, Z19, and Z21, were detected, the sensitivity and specificity of the model for thyroid tumor were 96% and 91%, respectively.

When the imprinted gene Z1 was added based on the three genes, the sensitivity and specificity of the model were 100% and 82%, respectively.

When the imprinted gene Z3 was added based on the three genes, the sensitivity and specificity of the model were 98% and 87%, respectively.

When the imprinted gene Z5 was added based on the three genes, the sensitivity and specificity of the model were 97% and 89%, respectively.

When the imprinted gene Z6 was added based on the three genes, the sensitivity and specificity of the model were 98% and 89%, respectively.

When the imprinted gene Z8 was added based on the three genes, the sensitivity and specificity of the model were 97% and 91%, respectively.

When the imprinted gene Z9 was added based on the three genes, the sensitivity and specificity of the model were 97% and 90%, respectively.

When the imprinted gene Z10 was added based on the three genes, the sensitivity and specificity of the model were 98% and 89%, respectively.

When the imprinted gene Z11 was added to three genes, the sensitivity and specificity of the model were 100% and 88%, respectively.

When the imprinted gene Z12 was added based on the three genes, the sensitivity and specificity of the model were 97% and 91%, respectively.

When the imprinted gene Z13 was added based on the three genes, the sensitivity and specificity of the model were 99% and 90%, respectively.

When the imprinted gene Z14 was added based on the three genes, the sensitivity and specificity of the model were 97% and 91%, respectively.

When the imprinted gene Z18 was added based on the three genes, the sensitivity and specificity of the model were 99% and 91%, respectively.

When the imprinted gene Z20 was added based on the three genes, the sensitivity and specificity of the model were 98% and 87%, respectively.

When the imprinted gene Z22 was added based on the three genes, the sensitivity and specificity of the model were 97% and 89%, respectively.

When the imprinted gene Z28 was added based on the three genes, the sensitivity and specificity of the model were 100% and 85%, respectively.

**Example 4: Verification of identification model of benign and malignant degree by imprinted genes in biopsy samples of benign and malignant lung tumors**

100 cases of fine needle aspiration samples of each of benign and malignant lung tumors were obtained and treated using the same method as described in Example 3. The benign and malignant degree of the tumor was graded using the model constructed in Example 2. The samples determined as benign tumor and cancer potential based on the benign and malignant degree of the tumor were recorded as benign samples, and the samples determined as early cancer, intermediate cancer, and terminal cancer based on the benign and malignant degree of the tumor were recorded as malignant samples. The results were compared with the benign and malignant degree diagnosed pathologically to evaluate the sensitivity and specificity of the model.

Table 7 shows the sensitivity and specificity of the models of the combination of imprinted genes Z16, Z19, and Z21 as well as the combination of imprinted gene Z1, Z3, Z5, Z6, Z8, Z9, Z10, Z11, Z12, Z13, Z14, Z18, Z20, Z22, or Z28 with Z16, Z19, and Z21, for detecting the benign and malignant degree of lung tumor.

[Table 7]

| Combination of imprinted genes | Sensitivity | Specificity |
|---|---|---|
| Z16+Z19+Z21 | 98% | 95% |
| Z16+Z19+Z21+Z1 | 100% | 91% |
| Z16+Z19+Z21+Z3 | 100% | 94% |
| Z16+Z19+Z21+Z5 | 100% | 93% |
| Z16+Z19+Z21+Z6 | 99% | 94% |
| Z16+Z19+Z21+Z8 | 99% | 95% |
| Z16+Z19+Z21+Z9 | 99% | 94% |
| Z16+Z19+Z21+Z10 | 99% | 93% |
| Z16+Z19+Z21+Z11 | 100% | 95% |
| Z16+Z19+Z21+Z12 | 99% | 94% |

(continued)

| Combination of imprinted genes | Sensitivity | Specificity |
|---|---|---|
| Z16+Z19+Z21+Z13 | 100% | 93% |
| Z16+Z19+Z21+Z14 | 99% | 95% |
| Z16+Z19+Z21+Z18 | 100% | 92% |
| Z16+Z19+Z21+Z20 | 100% | 93% |
| Z16+Z19+Z21+Z22 | 100% | 92% |
| Z16+Z19+Z21+Z28 | 100% | 90% |

As shown in Table 7, when only three imprinted genes, i.e., Z16, Z19, and Z21, were detected, the sensitivity and specificity of the model for lung tumors were 98% and 95%, respectively.

When the imprinted gene Z1 was added based on the three genes, the sensitivity and specificity of the model were 100% and 91%, respectively.

When the imprinted gene Z3 was added based on the three genes, the sensitivity and specificity of the model were 100% and 94%, respectively.

When the imprinted gene Z5 was added based on the three genes, the sensitivity and specificity of the model were 100% and 93%, respectively.

When the imprinted gene Z6 was added based on the three genes, the sensitivity and specificity of the model were 99% and 94%, respectively.

When the imprinted gene Z8 was added based on the three genes, the sensitivity and specificity of the model were 99% and 95%, respectively.

When the imprinted gene Z9 was added based on the three genes, the sensitivity and specificity of the model were 99% and 94%, respectively.

When the imprinted gene Z10 was added based on the three genes, the sensitivity and specificity of the model were 99% and 93%, respectively.

When the imprinted gene Z11 was added to three genes, the sensitivity and specificity of the model were 100% and 95%, respectively.

When the imprinted gene Z12 was added based on the three genes, the sensitivity and specificity of the model were 99% and 94%, respectively.

When the imprinted gene Z13 was added based on the three genes, the sensitivity and specificity of the model were 100% and 93%, respectively.

When the imprinted gene Z14 was added based on the three genes, the sensitivity and specificity of the model were 99% and 95%, respectively.

When the imprinted gene Z18 was added based on the three genes, the sensitivity and specificity of the model were 100% and 92%, respectively.

When the imprinted gene Z20 was added based on the three genes, the sensitivity and specificity of the model were 100% and 93%, respectively.

When the imprinted gene Z22 was added based on the three genes, the sensitivity and specificity of the model were 100% and 92%, respectively.

When the imprinted gene Z28 was added based on the three genes, the sensitivity and specificity of the model were 100% and 90%, respectively.

**Example 5: Verification of identification model of benign and malignant degree by imprinted genes in bladder tumor, prostate tumor, skin tumor, breast tumor, cervical tumor, intestinal tumor, stomach tumor, esophageal tumor, pancreatic tumor, and liver tumor**

Cystoscopic biopsy samples of benign and malignant bladder tumors, aspiration biopsy samples of benign and malignant prostate tumors, biopsy samples of benign and malignant skin tumors, aspiration biopsy samples of benign and malignant breast tumors, colposcopic biopsy samples of benign and malignant cervical tumors, enteroscopic biopsy samples of benign and malignant intestinal tumors, gastroscopic biopsy samples of benign and malignant stomach tumors, gastroscopic biopsy samples of benign and malignant esophageal tumors, aspiration biopsy samples of benign and malignant pancreatic tumors, and aspiration biopsy samples of benign and malignant liver tumors were obtained, with 100 cases for each benign or malignant pancreatic tumor. The samples were treated with the same method as described in Example 3. The expression statuses of imprinted genes Z16, Z19, Z21, Z1, Z3, Z5, Z6, Z8, Z9, Z10, Z11, Z12, Z13, Z14, Z18, Z20, Z22, and Z28 were detected, and TE, BAE, and MAE of each gene were quantified.

The benign and malignant degree of the tumor was graded using the model constructed in Example 2. The samples determined as benign tumor and cancer potential based on the benign and malignant degree of the tumor were recorded as benign samples, and the samples determined as early cancer, intermediate cancer, and terminal cancer based on the benign and malignant degree of the tumor were recorded as malignant samples. The results were compared with the benign and malignant degree diagnosed pathologically to evaluate the sensitivity and specificity of the model.

Tables 8 and 9 show the sensitivity and specificity of the models of the combination of imprinted genes Z16, Z19, and Z21 as well as the combination of imprinted gene Z1, Z3, Z5, Z6, Z8, Z9, Z10, Z11, Z12, Z13, Z14, Z18, Z20, Z22, or Z28 with Z16, Z19, and Z21, for detecting bladder tumor, prostate tumor, skin tumor, breast tumor, cervical tumor, intestinal tumor, stomach tumor, esophageal tumor, pancreatic tumor, and liver tumor.

Table 8

| | Bladder | | Prostate | | Skin | | Breast | | Cervical | |
|---|---|---|---|---|---|---|---|---|---|---|
| | Sensitivity | Specificity | Sensitivity | Specificity | Sensitivity | Specificity | Sensitivity | Specificity | Sensitivity | Specificity |
| Z16+Z19+Z21 | 96% | 97% | 94% | 97% | 93% | 92% | 94% | 96% | 97% | 93% |
| Z16+Z19+Z21+Z1 | 98% | 92% | 98% | 93% | 100% | 89% | 98% | 93% | 99% | 90% |
| Z16+Z19+Z21+Z3 | 100% | 96% | 96% | 94% | 95% | 90% | 97% | 94% | 99% | 91% |
| Z16+Z19+Z21+Z8 | 97% | 95% | 96% | 94% | 100% | 89% | 95% | 94% | 98% | 90% |
| Z16+Z19+Z21+Z10 | 98% | 94% | 99% | 94% | 94% | 89% | 99% | 94% | 99% | 91% |
| Z16+Z19+Z21+Z11 | 98% | 96% | 97% | 95% | 97% | 90% | 96% | 93% | 98% | 91% |
| Z16+Z19+Z21+Z13 | 99% | 94% | 96% | 95% | 100% | 90% | 98% | 93% | 99% | 91% |
| Z16+Z19+Z21+Z28 | 98% | 94% | 96% | 93% | 95% | 89% | 97% | 92% | 100% | 90% |

Table 9

| | Intestinal | | Stomach | | Esophageal | | Pancreas | | Liver | |
|---|---|---|---|---|---|---|---|---|---|---|
| | Sensitivity | Specificity | Sensitivity | Specificity | Sensitivity | Specificity | Sensitivity | Specificity | Sensitivity | Specificity |
| 216+219+221 | 94% | 94% | 96% | 92% | 96% | 94% | 98% | 92% | 97% | 90% |
| Z16+Z19+Z21+Z1 | 99% | 91% | 99% | 89% | 98% | 91% | 100% | 90% | 100% | 86% |
| Z16+Z19+Z21+Z3 | 98% | 92% | 98% | 90% | 97% | 92% | 100% | 91% | 100% | 88% |
| Z16+Z19+Z21+Z8 | 99% | 92% | 97% | 90% | 97% | 93% | 100% | 91% | 99% | 87% |
| Z16+Z19+Z21+Z10 | 100% | 92% | 100% | 90% | 97% | 91% | 100% | 90% | 100% | 87% |
| Z16+Z19+Z21+Z11 | 98% | 93% | 98% | 91% | 98% | 92% | 100% | 92% | 100% | 89% |
| Z16+Z19+Z21+Z13 | 99% | 92% | 99% | 91% | 100% | 92% | 99% | 89% | 100% | 87% |
| Z16+Z19+Z21+Z28 | 97% | 90% | 98% | 89% | 98% | 90% | 99% | 90% | 99% | 86% |

As shown in Tables 8 and 9, when three imprinted genes Z16, Z19, and Z21 were detected, the sensitivity and specificity of the model for detecting bladder cancer were 96% and 97%, respectively; the sensitivity and specificity of the model for detecting prostate cancer were 94% and 97%, respectively; the sensitivity and specificity of the model for detecting skin cancer were 93% and 92%, respectively; the sensitivity and specificity of the model for detecting breast cancer were 94% and 96%, respectively; the sensitivity and specificity of the model for detecting cervical cancer were 97% and 93%, respectively; the sensitivity and specificity of the model for detecting intestinal cancer were 94% and 94%, respectively; the sensitivity and specificity of the model for detecting stomach cancer were 96% and 92%, respectively; the sensitivity and specificity of the model for detecting esophageal cancer were 96% and 94%, respectively; the sensitivity and specificity of the model for detecting pancreatic cancer were 98% and 92%, respectively; and the sensitivity and specificity of the model for detecting liver cancer were 97% and 90%, respectively.

When imprinted gene Z1 was added based on the three genes, for detecting bladder tumor, the sensitivity was increased to 98%, and the specificity was 92%; for detecting prostate cancer, the sensitivity was increased to 98%, and the specificity was 93%; for detecting skin cancer, the sensitivity was increased to 100%, and the specificity was 89%; for detecting breast cancer, the sensitivity was increased to 98%, and the specificity was 93%; for detecting cervical cancer, the sensitivity was increased to 99%, and the specificity was 90%; for detecting intestinal cancer, the sensitivity was increased to 99%, and the specificity was 91%; for detecting stomach cancer, the sensitivity was increased to 99%, and the specificity was 89%; for detecting esophageal cancer, the sensitivity was increased to 98%, and the specificity was 91%; for detecting pancreatic cancer, the sensitivity was increased to 100%, and the specificity was 90%; and for detecting liver cancer, the sensitivity was increased to 100%, and the specificity was 86%.

When imprinted gene Z3 was added based on the three genes, for detecting bladder tumor, the sensitivity was increased to 100%, and the specificity was 96%; for detecting prostate cancer, the sensitivity was increased to 96%, and the specificity was 94%; for detecting skin cancer, the sensitivity was increased to 95%, and the specificity was 90%; for detecting breast cancer, the sensitivity was increased to 97%, and the specificity was 94%; for detecting cervical cancer, the sensitivity was increased to 99%, and the specificity was 91%; for detecting intestinal cancer, the sensitivity was increased to 98%, and the specificity was 92%; for detecting stomach cancer, the sensitivity was increased to 98%, and the specificity was 90%; for detecting esophageal cancer, the sensitivity was increased to 97%, and the specificity was 92%; for detecting pancreatic cancer, the sensitivity was increased to 100%, and the specificity was 91%; and for detecting liver cancer, the sensitivity was increased to 100%, and the specificity was 88%.

When imprinted gene Z8 was added based on the three genes, for detecting bladder tumor, the sensitivity was increased to 97%, and the specificity was 95%; for detecting prostate cancer, the sensitivity was increased to 96%, and the specificity was 94%; for detecting skin cancer, the sensitivity was increased to 100%, and the specificity was 89%; for detecting breast cancer, the sensitivity was increased to 95%, and the specificity was 94%; for detecting cervical cancer, the sensitivity was increased to 98%, and the specificity was 90%; for detecting intestinal cancer, the sensitivity was increased to 99%, and the specificity was 92%; for detecting stomach cancer, the sensitivity was increased to 97%, and the specificity was 90%; for detecting esophageal cancer, the sensitivity was increased to 97%, and the specificity was 93%; for detecting pancreatic cancer, the sensitivity was increased to 100%, and the specificity was 91%; and for detecting liver cancer, the sensitivity was increased to 99%, and the specificity was 87%.

When imprinted gene Z10 was added based on the three genes, for detecting bladder tumor, the sensitivity was increased to 98%, and the specificity was 94%; for detecting prostate cancer, the sensitivity was increased to 99%, and the specificity was 94%; for detecting skin cancer, the sensitivity was increased to 94%, and the specificity was 89%; for detecting breast cancer, the sensitivity was increased to 99%, and the specificity was 94%; for detecting cervical cancer, the sensitivity was increased to 99%, and the specificity was 91%; for detecting intestinal cancer, the sensitivity was increased to 100%, and the specificity was 92%; for detecting stomach cancer, the sensitivity was increased to 100%, and the specificity was 90%; for detecting esophageal cancer, the sensitivity was increased to 97%, and the specificity was 91%; for detecting pancreatic cancer, the sensitivity was increased to 100%, and the specificity was 90%; and for detecting liver cancer, the sensitivity was increased to 100%, and the specificity was 87%.

When imprinted gene Z11 was added based on the three genes, for detecting bladder tumor, the sensitivity was increased to 98%, and the specificity was 96%; for detecting prostate cancer, the sensitivity was increased to 97%, and the specificity was 95%; for detecting skin cancer, the sensitivity was increased to 97%, and the specificity was 90%; for detecting breast cancer, the sensitivity was increased to 96%, and the specificity was 93%; for detecting cervical cancer, the sensitivity was increased to 98%, and the specificity was 91%; for detecting intestinal cancer, the sensitivity was increased to 98%, and the specificity was 93%; for detecting stomach cancer, the sensitivity was increased to 98%, and the specificity was 91%; for detecting esophageal cancer, the sensitivity was increased to 98%, and the specificity was 92%; for detecting pancreatic cancer, the sensitivity was increased to 100%, and the specificity was 92%; and for detecting liver cancer, the sensitivity was increased to 100%, and the specificity was 89%.

When imprinted gene Z13 was added based on the three genes, for detecting bladder tumor, the sensitivity was increased to 99%, and the specificity was 94%; for detecting prostate cancer, the sensitivity was increased to 96%, and the specificity was 95%; for detecting skin cancer, the sensitivity was increased to 100%, and the specificity was 90%; for detecting breast cancer, the sensitivity was increased to 98%, and the specificity was 93%; for detecting cervical cancer, the sensitivity was increased to 99%, and the specificity was 91%; for detecting intestinal cancer, the sensitivity was increased to 99%, and the specificity was 92%; for detecting stomach cancer, the sensitivity was increased to 99%, and the specificity was 91%; for detecting esophageal cancer, the sensitivity was increased to 100%, and the specificity was 92%; for detecting pancreatic cancer, the sensitivity was increased to 99%, and the specificity was 89%; and for detecting liver cancer, the sensitivity was increased to 100%, and the specificity was 87%.

When imprinted gene Z28 was added based on the three genes, for detecting bladder tumor, the sensitivity was increased to 98%, and the specificity was 94%; for detecting prostate cancer, the sensitivity was increased to 96%, and the specificity was 93%; for detecting skin cancer, the sensitivity was increased to 95%, and the specificity was 89%; for detecting breast cancer, the sensitivity was increased to 97%, and the specificity was 92%; for detecting cervical cancer, the sensitivity was increased to 100%, and the specificity was 90%; for detecting intestinal cancer, the sensitivity was increased to 97%, and the specificity was 90%; for detecting stomach cancer, the sensitivity was increased to 98%, and the specificity was 89%; for detecting esophageal cancer, the sensitivity was increased to 98%, and the specificity was 90%; for detecting pancreatic cancer, the sensitivity was increased to 99%, and the specificity was 90%; and for detecting liver cancer, the sensitivity was increased to 99%, and the specificity was 86%.

### Example 6: Analysis of tumor lymphatic metastasis

Fine needle aspiration samples of sentinel lymph nodes of metastatic and non-metastatic breast cancers were obtained and treated using the same method as described in Example 2. The expression statuses of imprinted genes Z1, Z8, Z11, Z13, Z16, Z19, and Z21 were detected, and TE, BAE, and MAE of each gene were quantified.

The quantitative results are shown in Fig. 5 (see Figs. 5(a)-(g)). TE, BAE, and MAE of the imprinted genes Z1, Z8, Z11, Z13, Z16, Z19, and Z21 were significantly different in lymph nodes with and without tumor metastasis.

### Example 7: Analysis of expression statuses of BRAF and MYC genes in benign and malignant thyroid tumors

Fine needle aspiration samples of benign and malignant thyroid tumors were obtained and treated using the same method as described in Example 2. The expression of non-imprinted genes BRAF and MYC were detected. The BRAF and MYC genes detected using intron probes were quantified in the same manner as imprinted genes. Mutations in the BRAF gene detected using mRNA probes were quantified by counting 1,200 to 2,000 cells in which cells with more than 3 signal points were positive and calculating the proportion of the positive cells.

The quantitative results are shown in Fig. 6. Multi-allelic gene expression (MAE) of the BRAF and MYC genes and the proportion of BRAF mutant positive cells were significantly different in benign and malignant thyroid tumors.

### Example 8: Analysis of expression statuses of CDKN2A, HER2, and MYC genes in benign and malignant lung tumors

Fine needle aspiration samples of benign and malignant lung tumors were obtained and treated using the same method as described in Example 2. The expression of non-imprinted genes EGFR, HER2, and MYC were detected. The EGFR, HER2, and MYC genes were quantified in the same manner as imprinted genes.

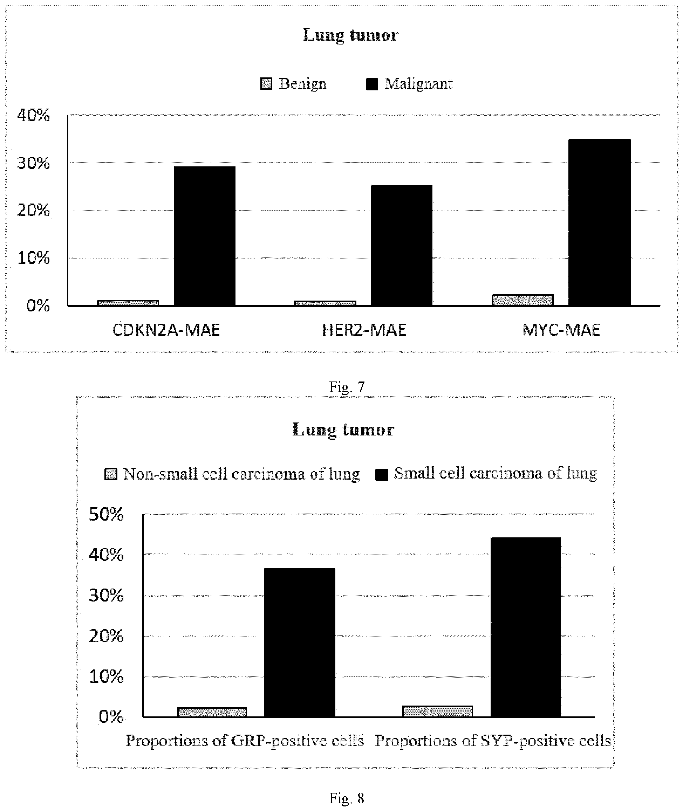

The quantitative results are shown in Fig. 7. Multi-allelic gene expression (MAE) of the EGFR, HER2, and MYC genes were significantly different in benign and malignant lung tumors.

### Example 9: Analysis of the expression statuses of GRP and SYP genes in small cell carcinoma of lung and non-small cell carcinoma of lung

Fine needle aspiration samples of small cell carcinoma of lung and non-small cell carcinoma of lung were obtained and treated using the same method as described in Example 2. The expression of non-imprinted genes GRP and SYP were detected. GRP and SYP were quantified by counting 1,200 to 2,000 cells in which cells with more than 2 signal points were positive and calculating the proportion of the positive cells.

The quantitative results are shown in Fig. 8. The proportions of GRP and SYP-positive cells were significantly different in small cell carcinoma of lung and non-small cell carcinoma of lung. Therefore, by adding the GRP and SYP genes on the basis of the detection of the expression statuses of imprinted genes, lung cancer was graded while achieving an early diagnosis of lung cancer, which is helpful for accurate treatment.

### Example 10: Analysis of expression statuses of CDKN2A and MYC genes in bladder cancer

Biopsy samples of benign and malignant bladder tumors were obtained and treated using the same method as described in Example 2. The expression of non-imprinted genes CDKN2A and MYC were detected. The CDKN2A and MYC genes were quantified in the same manner as imprinted genes.

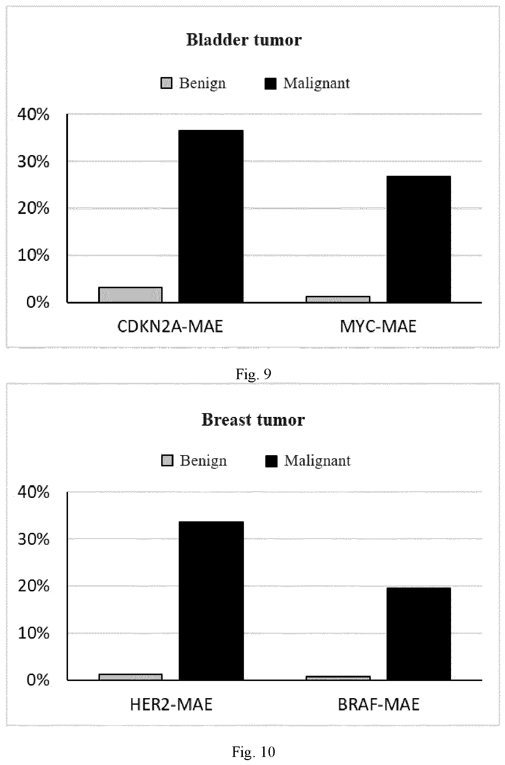

The quantitative results are shown in Fig. 9. Multi-allelic gene expression (MAE) of the CDKN2A and MYC genes were significantly different in benign and malignant bladder tumors.

**Example 11: Analysis of expression statuses of HER2 and BRAF genes in breast cancer**

Biopsy samples of benign and malignant breast tumors were obtained and treated using the same method as described in Example 2. The expression of non-imprinted genes HER2 and BRAF were detected. The HER2 and BRAF genes were quantified in the same manner as imprinted genes.

The quantitative results are shown in Fig. 10. Multi-allelic gene expression (MAE) of the HER2 and BRAF genes were significantly different in benign and malignant breast tumors.

In the description of the specification, references to descriptions of the terms "an embodiment", "some embodiments", "example", "specific example", or "some examples", etc. mean that a specific feature, structure, material, or characteristic described in connection with the embodiment or example are included in at least one embodiment or example of the present disclosure. In the specification, the schematic description of the above terms is unnecessarily against the same embodiment or example. Moreover, the specific feature, structure, material, or characteristic described may be combined in any suitable manner in any one or more embodiments or examples. In addition, without mutual contradiction, those skilled in the art may incorporate and combine different embodiments or examples and features of the different embodiments or examples described in the specification.

Although the embodiments of the present disclosure have been illustrated and described above, it should be understood that the above embodiments are illustrative and cannot be construed as limitations on the present disclosure. Changes, modifications, replacements, and variants may be made by those skilled in the art without departing from the scope of the present disclosure.

**Claims**

1. A grading model of gene expression status for a tumor, the model comprising:
   grading an expression status of imprinted genes by calculating changes in a total expression, single-allelic expression, bi-allelic expression, and multi-allelic expression of the imprinted genes in the tumor, wherein:

   the imprinted genes comprise an imprinted gene Z16, an imprinted gene Z19, and an imprinted gene Z21;
   the imprinted gene Z16 is SNRPN/SNURF;
   the imprinted gene Z19 is HM13; and
   the imprinted gene Z21 is SNU13.

2. The grading model according to claim 1, wherein the imprinted genes further comprise any one or more of an imprinted gene Z1, an imprinted gene Z3, an imprinted gene Z5, an imprinted gene Z6, an imprinted gene Z8, an imprinted gene Z9, an imprinted gene Z10, an imprinted gene Z11, an imprinted gene Z12, an imprinted gene Z13, an imprinted gene Z14, an imprinted gene Z18, an imprinted gene Z20, an imprinted gene Z22, or an imprinted gene Z28, wherein:

   the imprinted gene Z1 is GNAS;
   the imprinted gene Z3 is PEG10;
   the imprinted gene Z5 is MEST;
   the imprinted gene Z6 is PLAGL1;
   the imprinted gene Z8 is DCN;
   the imprinted gene Z9 is DLK1;
   the imprinted gene Z10 is GATM;
   the imprinted gene Z11 is GRB10;
   the imprinted gene Z12 is PEG3;
   the imprinted gene Z13 is SGCE;
   the imprinted gene Z14 is SLC38A4;
   the imprinted gene Z18 is GATA3;
   the imprinted gene Z20 is KCNQ1;
   the imprinted gene Z22 is PON2; and
   the imprinted gene Z28 is ZIC1.

3. The grading model according to claim 1, wherein formulas for calculating the total expression, single-allelic expression, bi-allelic expression, and multi-allelic expression of the imprinted genes are as follows:

$$\text{the total expression} = (b+c+d)/(a+b+c+d);$$

$$\text{the single-allelic expression} = b/(b+c+d);$$

$$\text{the bi-allelic expression} = c/(b+c+d);$$

and

$$\text{the multi-allelic expression} = d/(b+c+d),$$

where:

a denotes the number of cells in which no marker is present in a cell nucleus and the imprinted genes are not expressed;
b denotes the number of cells in which one imprinted gene marker is present in the cell nucleus;
c denotes the number of cells in which two imprinted gene markers are present in the cell nucleus; and
d denotes the number of cells in which more than two imprinted gene markers are present in the cell nucleus.

4. The grading model according to claim 1, wherein a bi-allelic expression of the imprinted gene Z16 is divided into five different grades:

- Grade 0: the bi-allelic expression of the imprinted gene Z16 is lower than 15%;
- Grade I: the bi-allelic expression of the imprinted gene Z16 ranges from 15% to 20%;
- Grade II: the bi-allelic expression of the imprinted gene Z16 ranges from 20% to 26%;
- Grade III: the bi-allelic expression of the imprinted gene Z16 ranges from 26% to 30%; and
- Grade IV: the bi-allelic expression of the imprinted gene Z16 is greater than 30%,

wherein a multi-allelic expression of the imprinted gene Z16 is divided into five different grades:

- Grade 0: the multi-allelic expression of the imprinted gene Z16 is lower than 1.4%;
- Grade I: the multi-allelic expression of the imprinted gene Z16 ranges from 1.4% to 3.4%;
- Grade II: the multi-allelic expression of the imprinted gene Z16 ranges from 3.4% to 5.5%;
- Grade III: the multi-allelic expression of the imprinted gene Z16 ranges from 5.5% to 9%; and
- Grade IV: the multi-allelic expression of the imprinted gene Z16 is greater than 9%, and

wherein a total expression of the imprinted gene Z16 is divided into two different grades:

- Grade 0: the total expression of the imprinted gene Z16 is lower than 17%; and
- Grade I: the total expression of the imprinted gene Z16 is greater than or equal to 17%.

5. The grading model according to claim 1, wherein a bi-allelic expression of the imprinted gene Z19 is divided into five different grades:

- Grade 0: the bi-allelic expression of the imprinted gene Z19 is lower than 12%;
- Grade I: the bi-allelic expression of the imprinted gene Z19 ranges from 12% to 20%;
- Grade II: the bi-allelic expression of the imprinted gene Z19 ranges from 20% to 26%;
- Grade III: the bi-allelic expression of the imprinted gene Z19 ranges from 26% to 30%; and
- Grade IV: the bi-allelic expression of the imprinted gene Z19 is greater than 30%,

wherein a multi-allelic expression of the imprinted gene Z19 is divided into five different grades:

- Grade 0: the multi-allelic expression of the imprinted gene Z19 is lower than 1.4%;
- Grade I: the multi-allelic expression of the imprinted gene Z19 ranges from 1.4% to 3.4%;
- Grade II: the multi-allelic expression of the imprinted gene Z19 ranges from 3.4% to 6.3%;
- Grade III: the multi-allelic expression of the imprinted gene Z19 ranges from 6.3% to 9%; and
- Grade IV: the multi-allelic expression of the imprinted gene Z19 is greater than 9%, and

wherein a total expression of the imprinted gene Z19 is divided into two different grades:

- Grade 0: the total expression of the imprinted gene Z19 is lower than 11%; and
- Grade I: the total expression of the imprinted gene Z19 is greater than or equal to 11%.

6. The grading model according to claim 1, wherein a bi-allelic expression of the imprinted gene Z21 is divided into five different grades:

- Grade 0: the bi-allelic expression of the imprinted gene Z21 is lower than 12%;
- Grade I: the bi-allelic expression of the imprinted gene Z21 ranges from 12% to 16%;
- Grade II: the bi-allelic expression of the imprinted gene Z21 ranges from 16% to 20%;
- Grade III: the bi-allelic expression of the imprinted gene Z21 ranges from 20% to 25%; and
- Grade IV: the bi-allelic expression of the imprinted gene Z21 is greater than 25%,

wherein a multi-allelic expression of the imprinted gene Z21 is divided into five different grades:

- Grade 0: the multi-allelic expression of the imprinted gene Z21 is lower than 1.3%;
- Grade I: the multi-allelic expression of the imprinted gene Z21 ranges from 1.3% to 3.2%;
- Grade II: the multi-allelic expression of the imprinted gene Z21 ranges from 3.4% to 4.9%;
- Grade III: the multi-allelic expression of the imprinted gene Z21 ranges from 4.9% to 6.2%; and
- Grade IV: the multi-allelic expression of the imprinted gene Z21 is greater than 6.2%, and

wherein a total expression of the imprinted gene Z21 is divided into two different grades:

- Grade 0: the total expression of the imprinted gene Z21 is lower than 14%; and
- Grade I: the total expression of the imprinted gene Z21 is greater than or equal to 14%.

7. The grading model according to claim 1, wherein a positive degree of the imprinted genes Z16, Z19, and Z21 is divided into negative, positive potential, low positive, moderate positive, and high positive, wherein:

when the total expression of the imprinted gene is Grade 0, or when the total expression of the imprinted gene is Grade I as well as the bi-allelic expression and multi-allelic expression are both Grade 0, the positive degree of the imprinted gene is determined to be negative;
when the total expression of the imprinted gene is Grade I as well as at least one of the bi-allelic expression and multi-allelic expression is Grade I and both are not Grade II, the positive degree of the imprinted gene is determined to be positive potential;
when the total expression of the imprinted gene is Grade I as well as at least one of the bi-allelic expression and multi-allelic expression is Grade II and both are not Grade III, the positive degree of the imprinted gene is determined to be low positive;
when the total expression of the imprinted gene is Grade I as well as at least one of the bi-allelic expression and multi-allelic expression is Grade III and both are not Grade IV, the positive degree of the imprinted gene is determined to be moderate positive; and
when the total expression of the imprinted gene is Grade I as well as at least one of the bi-allelic expression and multi-allelic expression is Grade IV, the positive degree of the imprinted gene is determined to be high positive, and
wherein the positive degree of the imprinted genes Z16, Z19, and Z21 are independent.

8. The grading model according to claim 1, wherein a benign and malignant degree of the tumor is divided into benign tumor, cancer potential, early cancer, intermediate cancer, and terminal cancer, wherein:

when the positive degree of the imprinted genes Z16, Z19, and Z21 are all negative, or when the positive degree of no more than one of the imprinted genes Z16, Z19, and Z21 is positive potential, the benign and malignant degree of the tumor is determined to be benign tumor;

when the positive degree of at least two of the imprinted genes Z16, Z19, and Z21 are positive potential, or when the positive degree of no more than one of the imprinted genes Z16, Z19, and Z21 is low positive, the benign and malignant degree of the tumor is determined to be cancer potential;

when the positive degree of at least two of the imprinted genes Z16, Z19, and Z21 is low positive, or when the positive degree of no more than one of the imprinted genes Z16, Z19, and Z21 is moderate positive, the tumor is early cancer;

when the positive degree of at least two of the imprinted genes Z16, Z19, and Z21 are moderate positive, or when the positive degree of no more than one of the imprinted genes Z16, Z19, and Z21 is high positive, the benign and malignant degree of the tumor is determined to be intermediate cancer; and

when the positive degree of at least two of the imprinted genes Z16, Z19, and Z21 is high positive, the benign and malignant degree of the tumor is determined to be terminal cancer.

9. The grading model according to claim 2, wherein:

when a positive degree of the imprinted genes Z16, Z19, and Z21 are all negative or positive potential, or when the positive degree of no more than one of the imprinted genes Z16, Z19, and Z21 is low positive as well as a positive degree of the imprinted genes Z1, Z3, Z5, Z6, Z8, Z9, Z10, Z11, Z12, Z13, Z14, Z18, Z20, Z22, and Z28 are all negative, the benign and malignant degree of the tumor is determined to be a benign tumor; and

when the positive degree of no more than one of the imprinted genes Z16, Z19, and Z21 is low positive as well as the positive degree of at least one of the imprinted genes Z1, Z3, Z5, Z6, Z8, Z9, Z10, Z11, Z12, Z13, Z14, Z18, Z20, Z22, and Z28 is positive, or when the positive degree of at least two of the imprinted genes Z16, Z19, and Z21 is low positive, or when the positive degree of at least one of the imprinted genes Z16, Z19, and Z21 is moderate positive, the benign and malignant degree of the tumor is determined to be a malignant tumor.

10. The grading model according to claim 1, wherein the tumor is selected from thyroid cancer, lung cancer, bladder cancer, prostate cancer, skin cancer, breast cancer, cervical cancer, intestinal cancer, stomach cancer, esophageal cancer, pancreatic cancer, and liver cancer.

11. The grading model according to claim 1, further comprising:
grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes BRAF and/or MYC, wherein:

when a positive degree of the imprinted genes Z16, Z19, and Z21 are all positive potential or below, or when the positive degree of only one of the imprinted genes Z16, Z19, and Z21 is low positive or above and the positive degree of the others of the imprinted genes Z16, Z19, and Z21 is negative or positive potential as well as the non-imprinted genes BRAF and MYC are both negative, a benign and malignant degree of the tumor is determined to be a benign tumor; and

when the positive degree of only one of the imprinted genes Z16, Z19, and Z21 is low positive or above and the positive degree of the others of the imprinted genes Z16, Z19, and Z21 is negative or positive potential as well as at least one of the non-imprinted genes BRAF and MYC is positive, or when the positive degree of at least two of the imprinted genes Z16, Z19, and Z21 is low positive or above, the benign and malignant degree of the tumor is determined to be a malignant tumor,

wherein the non-imprinted genes BRAF and MYC being negative means that the expression of the non-imprinted genes BRAF and MYC in a test sample is not different from that in a normal sample, and

wherein the non-imprinted genes BRAF and MYC being positive means that the expression of the non-imprinted genes BRAF and MYC in the test sample is increased compared to that in the normal sample.

12. The grading model according to claim 1, further comprising:
grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes HER2 and/or MYC, wherein:

when a positive degree of the imprinted genes Z16, Z19, and Z21 are all positive potential or below, or when the positive degree of only one of the imprinted genes Z16, Z19, and Z21 is low positive or above and the positive degree of the others of the imprinted genes Z16, Z19, and Z21 is negative or positive potential as well as the non-imprinted genes HER2 and MYC are both negative, the benign and malignant degree of the tumor is determined to be a benign tumor; and

when the positive degree of only one of the imprinted genes Z16, Z19, and Z21 is low positive or above and the positive degree of the others of the imprinted genes Z16, Z19, and Z21 is negative or positive potential as well as at least one of the non-imprinted genes HER2 and MYC is positive, or when the positive degree of at least two of the imprinted genes Z16, Z19, and Z21 is low positive or above, the benign and malignant degree of the tumor is determined to be a malignant tumor,

wherein the non-imprinted genes HER2 and MYC being negative means that the expression of the non-imprinted genes HER2 and MYC in a test sample is not different from that in a normal sample, and

wherein the non-imprinted genes HER2 and MYC being positive means that the expression of the non-imprinted genes HER2 and MYC in the test sample is increased compared to that in the normal sample.

13. The grading model according to claim 1, further comprising:
grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes GRP and/or SYP, wherein:

when a benign and malignant degree of the tumor is determined to be an early cancer or above in combination with the imprinted genes Z16, Z19, and Z21, and when at least one of the non-imprinted genes GRP and SYP is positive, a type of the tumor is determined to be small cell carcinoma; and

when the benign and malignant degree of the tumor is determined to be early cancer or above in combination with the imprinted genes Z16, Z19, and Z21, and when the non-imprinted genes GRP and SYP are both negative, the type of the tumor is determined to be non-small cell carcinoma,

wherein the non-imprinted genes GRP and SYP being negative means that the expression of the non-imprinted genes GRP and SYP in a test sample is not different from that in a normal sample, and

wherein the non-imprinted genes GRP and SYP being positive means that the expression of the non-imprinted genes GRP and SYP in the test sample is increased compared to that in the normal sample.

14. The grading model according to claim 1, further comprising:
grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes CDKN2A and/or MYC, wherein:

when a positive degree of the imprinted genes Z16, Z19, and Z21 are all positive potential or below, or when the positive degree of only one of the imprinted genes Z16, Z19, and Z21 is low positive or above and the positive degree of the others of the imprinted genes Z16, Z19, and Z21 is negative or positive potential as well as the non-imprinted genes CDKN2A and MYC are both negative, a benign and malignant degree of the tumor is determined to be a benign tumor; and

when the positive degree of only one of the imprinted genes Z16, Z19, and Z21 is low positive or above and the positive degree of the others of the imprinted genes Z16, Z19, and Z21 is negative or positive potential as well as at least one of the non-imprinted genes CDKN2A and MYC is positive, or when the positive degree of at least two of the imprinted genes Z16, Z19, and Z21 is low positive or above, the benign and malignant degree of the tumor is determined to be a malignant tumor,

wherein the non-imprinted genes CDKN2A and MYC being negative means that the expression of the non-imprinted genes CDKN2A and MYC in a test sample is not different from that in a normal sample, and

wherein the non-imprinted genes CDKN2A and MYC being positive means that the expression of the non-imprinted genes CDKN2A and MYC in the test sample is increased compared to that in the normal sample.

15. The grading model according to claim 1, further comprising:
grading the expression status of the imprinted genes and an expression status of non-imprinted genes by calculating the changes in the total expression, single-allelic expression, bi-allelic expression, and multi-allelic expression of the imprinted genes in the tumor as well as an expression of non-imprinted genes HER2 and/or BRAF, wherein:

when a positive degree of the imprinted genes Z16, Z19, and Z21 are all positive potential or below, or when the positive degree of only one of the imprinted genes Z16, Z19, and Z21 is low positive or above and the positive degree of the others of the imprinted genes Z16, Z19, and Z21 is negative or positive potential as well as the non-imprinted genes HER2 and BRAF are both negative, a benign and malignant degree of the tumor is determined to be a benign tumor; and

when the positive degree of only one of the imprinted genes Z16, Z19, and Z21 is low positive or above and the positive degree of the others of the imprinted genes Z16, Z19, and Z21 is negative or positive potential as well as at least one of the non-imprinted genes HER2 and BRAF is positive, or when the positive degree of at least two of the imprinted genes Z16, Z19, and Z21 is low positive or above, the benign and malignant degree of the tumor is determined to be a malignant tumor,

wherein the non-imprinted genes HER2 and BRAF being negative means that the expression of the non-imprinted genes HER2 and BRAF in a test sample is not different from that in a normal sample, and

wherein the non-imprinted genes HER2 and BRAF being positive means that the expression of the non-imprinted genes HER2 and BRAF in the test sample is increased compared to that in the normal sample.

16. Use of the grading model according to any one of claims 1 to 15 in the detection and/or treatment of tumors.

17. A method for detecting a tumor, comprising:
    determining a type and/or a benign and malignant degree of the tumor in a test sample using the grading model according to any one of claims 1 to 15.

18. The method according to claim 17, wherein the test sample is derived from a patient having or suspected of having a tumor selected from types of thyroid cancer, lung cancer, bladder cancer, prostate cancer, skin cancer, breast cancer, cervical cancer, intestinal cancer, stomach cancer, esophageal cancer, pancreatic cancer, and liver cancer.

19. A method for monitoring a tumor, comprising:
    determining, prior to and subsequent to medicament administration, a benign and malignant degree of the tumor in a patient using the grading model according to any one of claims 1 to 15, to monitor tumor development in the patient.

Fig. 1

Fig. 2(a)

Fig. 2(b)

Fig. 2(c)

Fig. 2(d)

Fig. 2(e)

Fig. 2(f)

Fig. 2(g)

Fig. 2(h)

Fig. 2(i)

Fig. 2(j)

Fig. 2(k)

Fig. 2(l)

Fig. 2(m)

Fig. 2(n)

Fig. 2(o)

Fig. 2(p)

Fig. 2(q)

Fig. 2(r)

Fig. 2(s)

Fig. 2(t)

Fig. 2(u)

Fig. 2(v)

Fig. 2(w)

Fig. 2(x)

Fig. 2(y)

Fig. 2(z)

Fig. 2(aa)

Fig. 3(a)

Fig. 3(b)

Fig. 3(c)

Fig. 3(d)

Fig. 3(e)

Fig. 3(f)

Fig. 3(g)

Fig. 3(h)

Fig. 3(i)

Fig. 3(j)

## Z10

Fig. 3(k)

## Z12

Fig. 3(l)

## Z13

Fig. 3(m)

## Z14

Fig. 3(n)

Fig. 3(o)

Fig. 3(p)

Fig. 3(q)

Fig. 3(r)

## Z2

BAE  AUC= 0.540
MAE  AUC= 0.546
TE    AUC= 0.529

Fig. 3(s)

## Z4

BAE  AUC= 0.590
MAE  AUC= 0.596
TE    AUC= 0.581

Fig. 3(t)

## Z15

BAE  AUC= 0.547
MAE  AUC= 0.594
TE    AUC= 0.596

Fig. 3(u)

## Z17

BAE  AUC= 0.501
MAE  AUC= 0.488
TE    AUC= 0.514

Fig. 3(v)

Fig. 3(w)

Fig. 3(x)

Fig. 3(y)

Fig. 3(z)

Fig. 3(aa)

Fig. 4(a)

Fig. 4(b)

Prediction of benign and malignant degree based on gene combination

Z1, Z11, Z16, Z19, Z21

N (high positive gene) = 2 → Terminal cancer

N (high positive gene) = 1
OR
N (moderate positive gene) = 2 → Intermediate cancer

N (moderate positive gene) = 1
OR
N (low positive gene) = 2 → Early cancer

N (low positive gene) = 1
OR
N (positive potential gene) = 2 → Cancer potential

Benign

Fig. 4(c)

Fig. 5(a)

Fig. 5(b)

Fig. 5(c)

Fig. 5(d)

Fig. 5(e)

Fig. 5(f)

Fig. 5(g)

Fig. 6

Fig. 7

Fig. 8

Fig. 9

Fig. 10

## INTERNATIONAL SEARCH REPORT

| International application No. |
|---|
| **PCT/CN2022/131540** |

| A. | CLASSIFICATION OF SUBJECT MATTER |
|---|---|

C12Q 1/6886(2018.01)i

According to International Patent Classification (IPC) or to both national classification and IPC

| B. | FIELDS SEARCHED |
|---|---|

Minimum documentation searched (classification system followed by classification symbols)

C12Q1/-

Documentation searched other than minimum documentation to the extent that such documents are included in the fields searched

Electronic data base consulted during the international search (name of data base and, where practicable, search terms used)

CNABS, CNTXT, WPI, EPODOC, CNKI: 立森印迹诊断, 基因, 诊断, 肿瘤, 程度, 模型, 缺失表达量, 拷贝数异常表达量, 变化, 分级, gene, diagnosis, tumor, degree, model, missing expression level, copy number abnormal expression level, change, grade

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 108977535 A (LISEN IMPRINTING DIAGNOSIS TECHNOLOGY (WUXI) CO., LTD.) 11 December 2018 (2018-12-11) description, paragraphs [0008]-[0095] | 1-19 |
| Y | WO 2021018116 A1 (LISEN IMPRINTING DIAGNOSIS TECHNOLOGY CO., LTD.) 04 February 2021 (2021-02-04) description, pages 1-15 | 1-19 |
| A | US 2013096021 A1 (CHINNAIYAN, Arul M. et al.) 18 April 2013 (2013-04-18) entire document | 1-19 |
| A | CN 111105842 A (LISEN IMPRINTING DIAGNOSIS TECHNOLOGY (WUXI) CO., LTD.) 05 May 2020 (2020-05-05) entire document | 1-19 |
| A | CN 111100930 A (LISEN IMPRINTING DIAGNOSIS TECHNOLOGY (WUXI) CO., LTD.) 05 May 2020 (2020-05-05) entire document | 1-19 |
| A | US 2016083791 A1 (PATHADVANTAGE ASSOCIATED) 24 March 2016 (2016-03-24) entire document | 1-19 |

☐ Further documents are listed in the continuation of Box C.   ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **16 January 2023** | **28 January 2023** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

<table>
<tr><td colspan="3" align="center">INTERNATIONAL SEARCH REPORT<br>Information on patent family members</td><td colspan="2">International application No.<br><br>**PCT/CN2022/131540**</td></tr>
</table>

| Patent document cited in search report | | | Publication date (day/month/year) | Patent family member(s) | | | Publication date (day/month/year) |
|---|---|---|---|---|---|---|---|
| CN | 108977535 | A | 11 December 2018 | WO | 2018218737 | A1 | 06 December 2018 |
| WO | 2021018116 | A1 | 04 February 2021 | EP | 4006171 | A1 | 01 June 2022 |
| | | | | US | 2022275459 | A1 | 01 September 2022 |
| | | | | JP | 2022542987 | A | 07 October 2022 |
| US | 2013096021 | A1 | 18 April 2013 | EP | 2761300 | A2 | 06 August 2014 |
| | | | | WO | 2013089882 | A2 | 20 June 2013 |
| CN | 111105842 | A | 05 May 2020 | | None | | |
| CN | 111100930 | A | 05 May 2020 | WO | 2020082642 | A1 | 30 April 2020 |
| US | 2016083791 | A1 | 24 March 2016 | | None | | |

Form PCT/ISA/210 (patent family annex) (January 2015)

**REFERENCES CITED IN THE DESCRIPTION**

**Patent documents cited in the description**

- CN 202111342507